# EUROPEAN PATENT SPECIFICATION

(11) **EP 3 601 002 B1**
(45) Date of publication and mention of the grant of the patent: **20.03.2024**
(21) Application number: 18771699.8
(22) Date of filing: 06.03.2018
(51) Int. Cl.: B60W 40/08, B60W 60/00

(54) **TOUCH SENSOR SYSTEMS AND METHODS FOR VEHICLES**
BERÜHRUNGSSENSORSYSTEME UND VERFAHREN FÜR FAHRZEUGE
SYSTÈMES ET PROCÉDÉS DE CAPTEUR TACTILE POUR VÉHICULES

(30) Priority: 20.03.2017 US 201762473524 P; 04.10.2017 US 201762567765 P
(43) Date of publication of application: 05.02.2020
(73) Proprietor: Neonode Inc., Wilmington DE 19801 (US)
(72) Inventor: FROJDH, Gunnar Martin, 13771 Dalaro (SE); JUBNER, Alexander, 16346 Spanga (SE); ERIKSSON, Bjorn Thomas, 114 32 Stockholm (SE); HOLMGREN, Stefan Johannes, 19277 Sollentuna (SE); SVERUD, Per Oscar, 17446 Sundbyberg (SE)
(74) Representative: Lavoix
(86) International application number: PCT/US2018/020989
(87) International publication number: WO 2018/175101

(56) References cited:
- EP-A1- 3 124 352
- DE-A1-102014 118 958
- DE-A1-102017 208 762
- US-A1- 2004 044 203
- US-A1- 2012 312 956
- US-A1- 2015 123 947
- US-A1- 2016 084 869
- US-A1- 2016 377 508
- US-A1- 2017 054 946

## Description

### FIELD OF THE INVENTION

The field of the present invention is driver user interfaces for vehicles, including (i) safety features that verify that a driver is gripping the steering wheel, (ii) safety features for advanced driver-assistance systems (ADAS) vehicles, and (iii) user interfaces for vehicle functions.

### BACKGROUND OF THE INVENTION

EP 3124352 A1 describes a vehicle system that detects traffic and other driving conditions when the vehicle is in automated driving mode and warns the driver to switch from automated driving mode to manual driving mode when the detected conditions warrant greater driving care. The warning is provided by illuminating portions of the vehicle steering wheel that the driver shall take hold of in manual driving mode.

US 2015/123947 A1 describes a vehicle steering wheel configured to receive input commands from a driver in the form of gestures performed in a notch along the perimeter of the steering wheel. Beneath the notch, an array of optical proximity sensors detects the gestures.

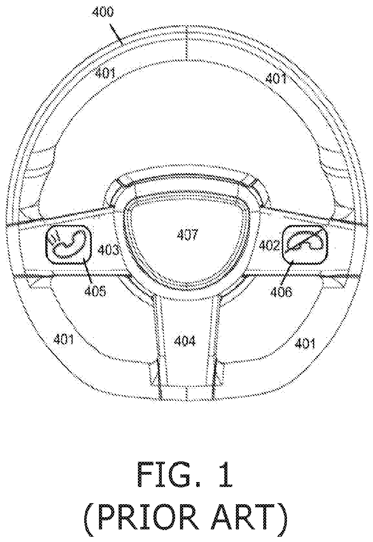

Reference is made to **FIG. 1**, which is a simplified illustration of a prior art steering wheel. A steering wheel **400**, shown in **FIG. 1**, includes a circular gripping member **401**, one or more connecting members **402** - **404** that connect the gripping member **401** to steering column **407**, and buttons **405** and **406** on connecting members **402** and **403** for controlling various devices in the vehicle. Connecting members **402** - **404**, which connect gripping member **401** to steering column **407**, are also referred to as spokes. In **FIG. 1**, button **405** is used to answer an incoming phone call on the vehicle's BLUETOOTH^{®} speaker phone and button **406** hangs up the call. BLUETOOTH is a trademark owned by the Bluetooth SIG of Kirkland, WA, USA. Controls mounted in a steering wheel can be operated comfortably and safely since the driver is able to control and operate these controls without taking hands off the wheel or eyes off the road.

Prior art cars are equipped with a safety device in the form of sensors in the steering wheel that detect whether the diver is gripping the wheel and issue a warning when the driver's hands are not on the wheel. The prior art sensors do not distinguish a human hand from any other object touching the wheel. This could lead certain drivers to trick the safety device by wrapping an object around the steering wheel grip when driving without holding on to the steering wheel.

ADAS vehicles require driver intervention in certain situations. It would thus be advantageous to track and measure driver preparedness for such intervention in ADAS vehicles.

In many safety-critical systems, such as those found in aircraft, certain controls are required to be duplicated. It would thus be advantageous to provide duplicated touch sensors for detecting touch and gestures in safety-critical systems.

In modern vehicles, the user interface for vehicle ignition is by pressing a button on the dashboard. It would be advantageous to provide user interfaces that are more user friendly and intuitive than a dashboard button.

### SUMMARY

There is provided in accordance with an embodiment of the present invention, a vehicle including an advanced driver-assistance system (ADAS) that selectively performs and selectively stops performing automated vehicle navigation operations, a steering wheel with which a human driver steers the vehicle when the automated vehicle navigation operations are not performed, a sensor operable to detect a driver's hand at a plurality of locations on the steering wheel, a plurality of visible-light emitters operable to illuminate the plurality of locations on the steering wheel, and a processor, coupled with the ADAS, with the sensor and with the visible-light emitters, performing the following functions while the ADAS is performing the automated vehicle navigation operations: (i) an activating function that activates at least one of the emitters to illuminate a location on the steering wheel, (ii) a detecting function that detects, via the sensor, when the driver touches the illuminated location, and (iii) a calculating function that calculates a degree of driver preparedness for taking control of steering the vehicle when the ADAS stops performing the automated vehicle navigation operations, based on at least one of time elapsed between the activating function and the detecting function, and proximity of the driver touch to the illuminated location. In some embodiments of the invention, the activating function causes an audio system in the vehicle to emit an audio prompt indicating that the driver should touch the illuminated location.

In some embodiments of the invention, the processor detects, via the sensor, an initial location of the driver's hand on the steering wheel prior to performing the activating function, and selects a location on the steering wheel to illuminate based on the detected initial location.

In some embodiments of the invention, the processor detects, via the sensor, initial locations of the driver's two hands on the steering wheel prior to performing the activating function, and further identifies the driver's handedness based on which hand touches the illuminated location.

In some embodiments of the invention, the ADAS configures a parameter of the automated vehicle navigation operations in response to the degree of driver preparedness calculated by the processor. The parameter is, e.g., a distance from a vehicle ahead or vehicle speed.

In some embodiments of the invention, the ADAS navigates the vehicle to a parking location in response to a low degree of driver preparedness calculated by the processor.

In some embodiments of the invention, the processor configures the activating function based on a prior calculated degree of driver preparedness.

In some embodiments of the invention, the activating function selects an illumination color based on the prior calculated degree of driver preparedness.

In some embodiments of the invention, the ADAS instructs the processor to perform the activating, detecting and calculating functions, based on an anticipated change in driving conditions.

In some embodiments of the invention, the anticipated change in driving conditions is an automated navigation instruction to exit a highway.

### BRIEF DESCRIPTION OF THE DRAWINGS

The present invention will be more fully understood and appreciated from the following detailed description, taken in conjunction with the drawings in which:
**FIG. 1** is a simplified illustration of a prior art steering wheel;
**FIG. 2** is an exploded view of a steering wheel, in accordance with an embodiment of the present invention;
**FIG. 3** is a cutaway view of a segment of the steering wheel of **FIG. 2**, in accordance with an embodiment of the present invention;
**FIGS. 4** and **5** are exploded views of the steering wheel segment illustrated in **FIG. 3**, in accordance with an embodiment of the present invention;
**FIG. 6** is a simplified illustration of electronic components in the steering wheel segment of **FIG. 3** connected to a processor, in accordance with an embodiment of the present invention;
**FIG. 7** is a simplified illustration of a structure of light baffles placed upon the electronic components in **FIG. 6**, in accordance with an embodiment of the present invention;
**FIG. 8** is a simplified illustration of light beams detecting an object, in accordance with an embodiment of the present invention;
**FIG. 9** is a simplified side view illustration of light beams projected radially outward from a steering wheel, in accordance with an embodiment of the present invention;
**FIG. 10** is a simplified illustration of communication between touch detection firmware and multiple clients over a network, in accordance with an embodiment of the present invention;
**FIGS. 11A** - **11E** are simplified illustrations of five basic gesture components used in a steering wheel user interface;
**FIG. 12** is a flowchart of an exemplary vehicle user interface;
**FIGS. 13A** - **13I** are simplified illustrations of user interface gestures performed on a steering wheel for an exemplary adaptive cruise control function;
**FIG. 14** is a simplified illustration of a multi-touch double-tap gesture to activate an autonomous drive mode;
**FIGS. 15A** and **15B** are simplified illustrations of a user interface indicating activation of an autonomous drive mode;
**FIGS. 16A** and **16B** are simplified illustrations of a gesture and an exemplary user interface for exiting autonomous drive mode;
**FIGS. 17A** and **17B** are simplified illustrations showing how an incoming call is received;
**FIGS. 18A** and **18B** are simplified illustrations showing how to hang up a call;
**FIGS. 19A** and **19B** are simplified illustrations of a user interface for a park assist function;
**FIGS. 20** - **26** are simplified illustrations of multi-finger pinch and stretch gestures;
**FIGS. 27** and **28** are simplified illustrations of multi-finger gestures;
**FIGS. 29** and **30** are simplified illustrations of one-finger gestures;
**FIGS. 31** - **34** are simplified illustrations of multi-finger gestures;
**FIG. 35** is a simplified illustration of micro-movements detected on a steering wheel;
**FIG. 36** is a simplified illustration of blood flow through a hand, the blood flow being detected by a reflectance-based sensor;
**FIGS. 37** - **44** are graphs of filtered detections of blood flow inside a hand gripping a steering wheel equipped with reflectance-based sensors;
**FIG. 45** is a pair of graphs, namely, a top graph of detections of blood flow inside a hand gripping a steering wheel, and a bottom graph of an estimated period in the detections of the top graph;
**FIG. 46** is a graph illustrating the spectrum of a pulse-detection signal;
**FIG. 47** is a graph illustrating modulation of a pulse-detection signal over time;
**FIG. 48** is a graph illustrating an estimated frequency of a pulse-detection signal;
**FIGS. 49A** - **49C** are simplified illustrations of a system for measuring driver alertness, in accordance with an embodiment of the present invention;
**FIG. 50** is a simplified illustration of a gesture for starting a vehicle; and
**FIGS. 51A, 51B****,** **52** and **53** are simplified illustrations of systems with duplicated touch sensors.

In the figures, the following numbering scheme is used. Light transmitters are numbered in the 100's, light detectors are numbered in the 200's, light guides and lenses are numbered in the 300's, miscellaneous items are numbered in the 400's, light beams are numbered in the 600's, and flow chart elements are numbered **1000** - **1100.** Like numbered elements are similar but not necessarily identical.

The following tables catalog the numbered elements and list the figures in which each numbered element appears.

| **Light Transmitters** | | | |
|---|---|---|---|
| **Element** | **Figures** | **Element** | **Figures** |
| **100** | 4, 5 | **107** | 6, 7 |
| **101** | 6 | **108** | 6, 7 |
| **102** | 6 | **109** | 6, 7 |
| **105** | 8, 9 | **110** - **119** | 53 |
| **106** | 6, 7 | | |

| **Light Detectors** | | | |
|---|---|---|---|
| **Element** | **Figures** | **Element** | **Figures** |
| **200** | **4** | **205** | **8** |
| **201** | **6, 8** | **206** | **8** |
| **202** | **6, 8** | **210-219** | **53** |
| **203** | **8** | | |

| **Light Guides and Lenses** | | | |
|---|---|---|---|
| | | | |
| **Element** | **Figures** | **Element** | **Figures** |
| **300** | **2 - 7** | **304** | **8** |
| **301** | **8** | **305** | **8, 9** |
| **302** | **8** | **310 - 319** | **53** |
| **303** | **8** | | |

| **Miscellaneous Items** | | |
|---|---|---|
| **Element** | **Figures** | **Description** |
| **400** | **1** | steering wheel |
| **401** | **1** | grip |
| **402** | **1** | right spoke |
| **403** | **1** | left spoke |
| **404** | **1** | bottom spoke |
| **405** | **1** | answer button |
| **406** | **1** | reject button |
| **407** | **1, 24 - 34** | steering wheel hub |
| **410** | **11, 13 - 34, 49 - 51** | steering wheel |
| **411** | **2 - 5, 52** | steering wheel frame |
| **412** | **2 - 5, 52** | top cover |
| **413** | **2 - 5, 52** | thumb notch |
| **414** | **2 - 7, 52** | PCB |
| **415** | **2 - 5, 7** | light baffle |
| **416** | **3, 5, 52** | transparent cover section |
| **417** | **3, 5, 52** | transparent cover section |
| **418** | **11, 14, 17, 18** | finger |
| **419** | **11, 13, 16 - 19, 35** | hand |
| **420** | **11, 14** | steering wheel surface |
| **421 - 424** | **11, 14** | hand/finger movement directions |
| **425** | **11** | clock icon |
| **426** | **11, 14** | finger |
| **428** | **13, 14** | hand/finger movement directions |
| **430** | **13** | double-tap gesture |
| **431 - 434** | **13** | hand/finger movement directions |
| **436** | **13, 15, 16** | Illumination |
| **437** | **13** | movement of illumination |
| **438** | **13, 17** | tap gesture |
| **440** | **6, 10** | processor |
| **441 - 443** | **10** | network client |
| **444** | **10** | message bus |
| **445 - 447** | **19** | icon |
| **450 - 459** | **20 - 34** | finger movement |
| **460, 461** | **24 - 34, 51** | touch pad |
| **465** | **35** | arrow |
| **466, 467** | **22, 26, 31 - 33** | stationary finger gesture |
| **468, 469** | **49, 50** | hand |
| **470, 471** | **49** | Indicator |
| **472, 473** | **50** | sweep gesture |
| **474, 475** | **51, 53** | one-dimensional sensor array |
| **476, 477** | **52, 53** | CPU |

| **Light Beams** | | |
|---|---|---|
| **Element** | **Figures** | **Description** |
| **601** | **9** | light beam |
| **602** | **3, 8, 9** | light beam |
| **603** | **8** | light beam |
| **604** | **8** | light beam |
| **605, 606** | **52** | light beam |

| **Flow Chart Stages** | | |
|---|---|---|
| **Element** | **Figures** | **Description** |
| **1001 - 1005** | **12** | vehicle application state |
| **1010 - 1019** | **12** | vehicle application action |

### DETAILED DESCRIPTION

Aspects of the present invention relate to light-based touch controls that allow a driver to keep his hands on a steering element while operating peripheral electronic devices and automated features in a vehicle.

A steering wheel is provided with a touch sensitive strip disposed along the entire circumference of the steering wheel. In order to facilitate locating the strip, it is disposed in a thumb receiving notch or groove that is etched or otherwise formed along the circumference of the steering wheel. In addition to a touch sensor, there is also a visible-light illuminator behind or around the touch sensitive strip that is used to indicate the state of the user interface to the user, and also indicate where certain tap gestures should be performed.

A user interface for this steering wheel is designed to be independent of the rotation of the steering wheel. Sweep gestures are clockwise and counter-clockwise so that they are independent of rotation of the wheel. A function is activated in response to a gesture, such as a double-tap, performed anywhere along the circumference of the wheel. The activation of some functions places the user interface into a state in which one or more additional functions can be selectively activated. In order to activate these additional functions, the touch location at which the initial gesture was performed is illuminated and subsequent gestures are performed in relation to the illuminated portion of the wheel. When a portion of the wheel is thus illuminated, and the driver slides his hand along the steering wheel grip, the illuminated portion of the steering wheel follows the hand so that the hand is always next to the location for performing subsequent gestures. Similarly, when the user switches hands gripping the steering wheel, the illumination jumps to the newly gripped part of the wheel.

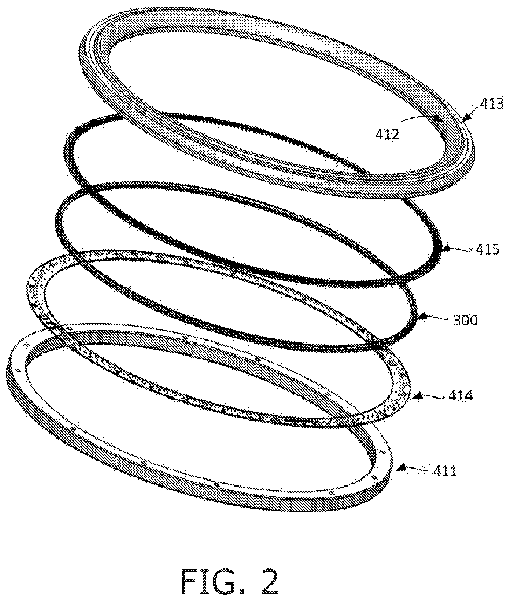

Reference is made to **FIG. 2**, which is an exploded view of a steering wheel, in accordance with an embodiment of the present invention. Elements of this steering wheel include steering wheel frame **411**, PCB **414**, an array of lenses **300**, a light baffle structure **415**, and a steering wheel top cover **412**. A thumb-receiving notch **413** is disposed within steering wheel cover **412**.

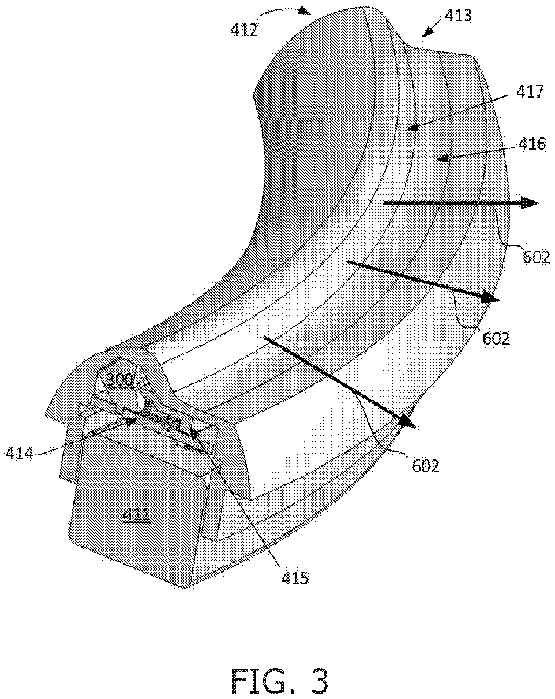

Reference is made to **FIG. 3**, which is a cutaway view of a segment of the steering wheel of **FIG. 2**, in accordance with an embodiment of the present invention. Thumb-receiving notch **413** is illustrated in **FIG. 3****.** Two light transmissive portions of cover **412** are also shown in **FIG. 3**. A first light transmissive portion **417** forms the side wall of thumb-receiving notch **413**. Light beams traveling into and out of this portion provide touch detection and proximity detection, as explained below. Three touch detection light beams **602** are shown directed radially outward from the steering wheel gripping element. The second light transmissive portion **416** forms a floor of thumb-receiving notch **413**, and is used for visible illumination indicating a state of the user interface to the driver, and at which location the driver should perform additional user interface gestures.

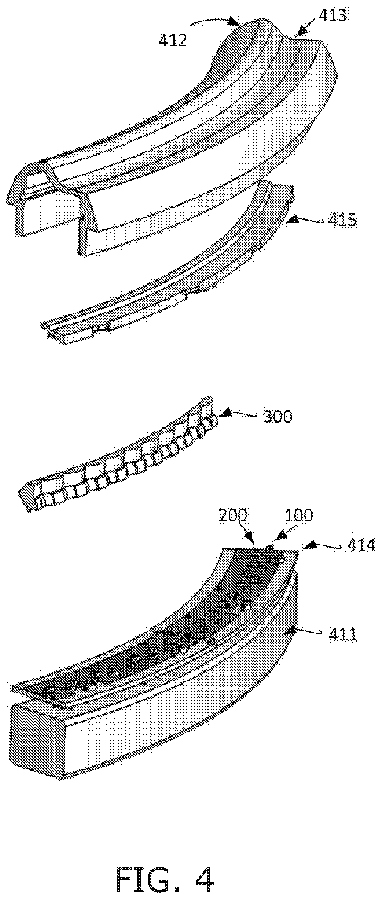

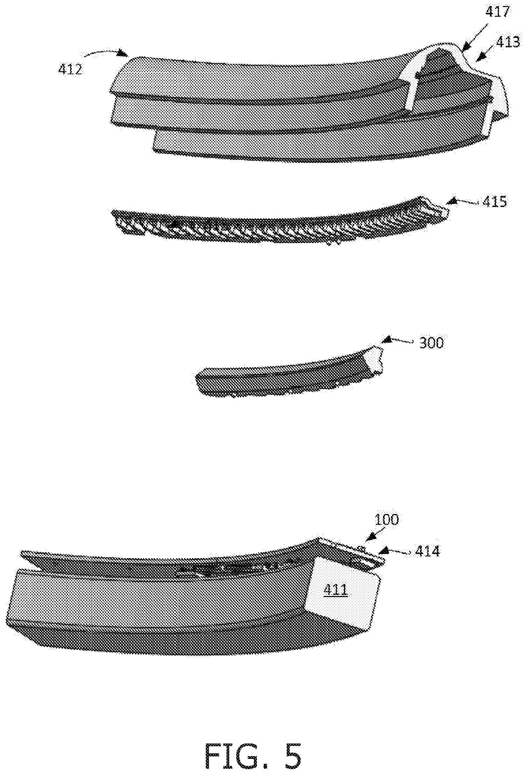

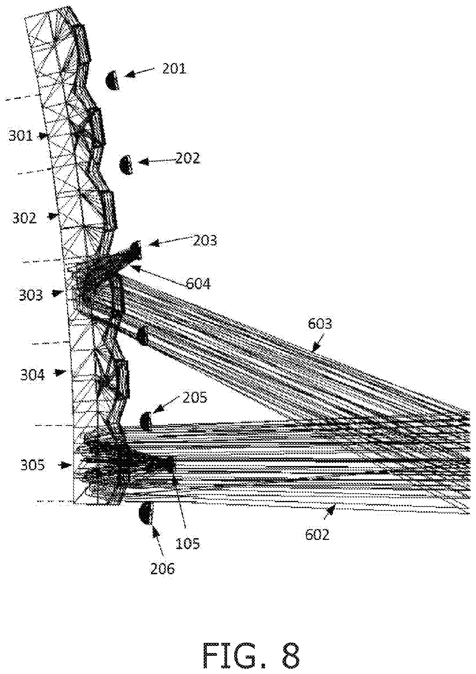

Reference is made to **FIGS. 4** and **5**, which are exploded views of the steering wheel segment illustrated in **FIG. 3**, in accordance with an embodiment of the present invention. As shown in **FIG. 4**, two concentric rows of elements are mounted on PCB **414**. Namely, an inner row of light detectors **200** and an outer row of light emitters **100**. Light from the emitters enters lenses **300** through which it is re-directed out of the steering wheel through light transmissive portion **417** as light beams **602**, illustrated in **FIGS. 3**, **8** and **9****.** An object such as a thumb placed in notch **413** reflects the light back through portion **417** and lenses **300** onto one or more of the light detectors **200**, thereby providing touch detection, as illustrated in **FIG. 8**. Similarly, an object such as a user's hand placed along the outer rim of the steering wheel outside notch **413** and opposite light transmissive portion **417** also reflects the light back through portion **417** and lenses **300** onto one or more of the light detectors **200**, thereby providing proximity detection.

**FIG. 5** shows an exploded view from below of the steering wheel segment illustrated in **FIG. 3**, in accordance with an embodiment of the present invention.

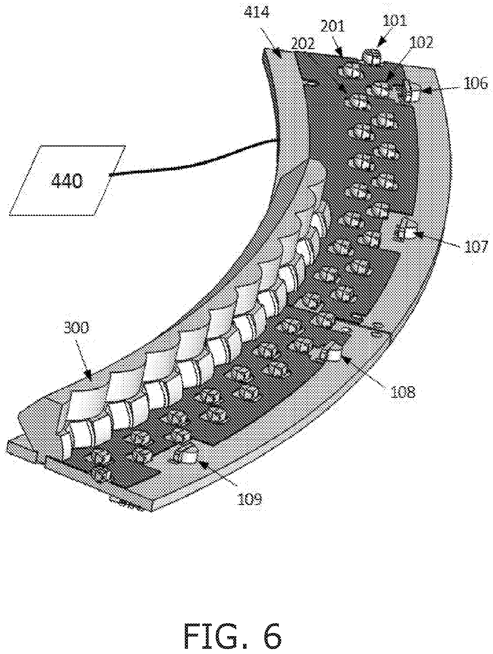

Reference is made to **FIG. 6**, which is a simplified illustration of electronic components in the steering wheel segment of **FIG. 3** connected to a processor, in accordance with an embodiment of the present invention. **FIG. 6** shows processor **440** connected to PCB **414** on which three concentric arrangements of light elements are mounted, namely, an inner circular arrangement of inward facing light detectors, including detectors **201** and **202**; a middle circular arrangement of inward facing light emitters, including emitters **101** and **102**; and an outer circular arrangement of outward facing light emitters **106** - **109.** The inward facing light emitters are used for touch and proximity detection and typically emit light in the near infrared range. Processor **440** controls activation of the emitters and detectors, and detects gestures performed on the steering wheel based on these activations and based on the outputs of the detectors.

The outward facing light emitters are used to provide visual indications to the user by illuminating light transmissive portion **416** of the steering wheel cover, and emit light in the visible range. Lenses **300** are described in U.S. Patent No. 9,741,184, entitled DOOR HANDLE WITH OPTICAL PROXIMITY SENSORS, the contents of which are incorporated herein in their entirety by reference.

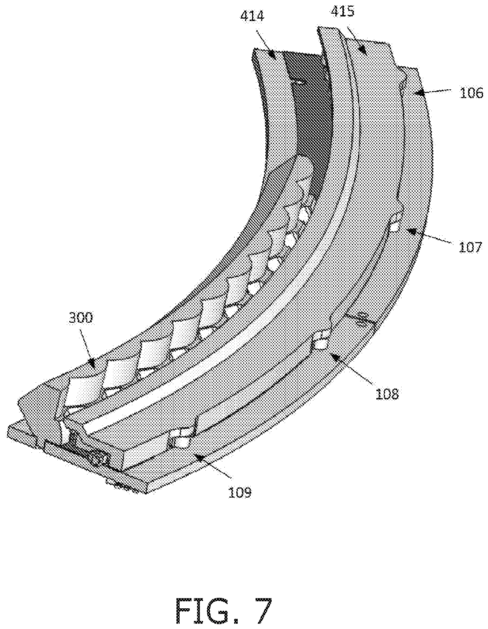

Reference is made to **FIG. 7**, which is a simplified illustration of a structure of light baffles placed upon the electronic components in **FIG. 6**, in accordance with an embodiment of the present invention. **FIG. 7** shows PCB **414** and lenses **300** of **FIG. 6**, but with baffle structure **415** placed above the mounted light elements.

Reference is made to **FIG**. **8**, which is a simplified illustration of light beams detecting an object, in accordance with an embodiment of the present invention. **FIG**. **8** illustrates a light path used to detect an object. Shown in **FIG**. **8** are individual lens structures **301** - **305.** Each lens structure serves a respective opposite emitter and two detectors, one to the left of the emitter and one to the right of the emitter. Thus, for example, lens structure **305** serves emitter **105** and detectors **205** and **206.** In addition, each detector is served by two lens structures; e.g., detector **205** receives reflected light from lens structures **304** and **305.** In the example shown in **FIG. 8**, light from emitter **105** is reflected by an object (not shown) into lens structure **303** and onto detector **203**. Three segments of the detected light are indicated in **FIG. 8**; namely, light beam 602 projected outward from lens structure **305** and radially outward of the steering wheel, light beam **603** reflected by the object into lens structure 303, and light beam **604** directed by lens structure **303** onto detector **203**.

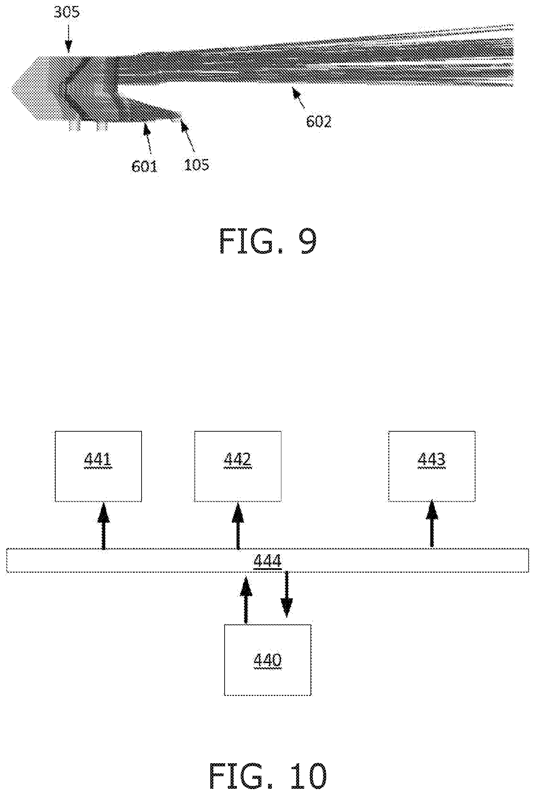

Reference is made to **FIG. 9**, which is a simplified side view illustration of light beams projected radially outward from a steering wheel, in accordance with an embodiment of the present invention. **FIG. 9** shows a cutaway side view of the light path illustrated in **FIG. 8****.** **FIG. 9** shows light beam **601** from emitter **105** entering lens structure **305**, where it is redirected outward as light beam **602**.

Methods for determining two-dimensional coordinates of an object detected by the disclosed proximity sensor array are described in U.S. Patent No. 9,164,625, entitled OPTICAL PROXIMITY SENSORS, and U.S. Patent No. 9,741,184, entitled DOOR HANDLE WITH OPTICAL PROXIMITY SENSORS, both incorporated herein in their entireties by reference. Because the present invention is a steering wheel and the proximity sensor array is arranged along an arc-shaped grip of the steering wheel, the determined coordinates are polar coordinates, including a polar angle and a radial coordinate. The polar angle corresponds to a coordinate along the proximity sensor array, which in US Patent Nos. 9,164,625 and 9,741,184 is described as an x-axis coordinate. The radial coordinate corresponds to a distance from the proximity sensor array, which in US Patent Nos. 9,164,625 and 9,741,184 is described as a y-axis coordinate.

Discussion now turns to the firmware and software used to detect and interpret user gestures. There are five basic gesture components that are detected by the hardware and low level drivers: *(i)* Thumb-Tap, *(ii)* Thumb-Glide, *(iii)* Thumb-Long-Press, *(iv)* Grab and *(v)* Rim-Tap. These components are emitted on the network as they are detected, and are used by higher level software to assemble more complex gestures such as double-taps. Application software interprets these gestures as input commands. In some cases multiple client applications are connected via a network to the detector firmware. The firmware sends information for each detected gesture component over the network, and a client application translates that information into commands and/or constructs compound gestures from multiple gesture components.

Reference is made to **FIG. 10**, which is a simplified illustration of communication between touch detection firmware and multiple clients over a network, in accordance with an embodiment of the present invention. **FIG. 10** shows an exemplary network architecture in which processor **440** sends detected gesture components over message bus **444**, e.g., using the Message Queue Telemetry Transport (MQTT) messaging protocol on top of the TCP/IP protocol, to connected clients **441** - **443.**

The five basic gesture components are categorized according to whether they are performed by a large object (hand) or small object (thumb), and whether the nature of the gesture component is discrete or continuous, as presented in the table below.

| **Component** | **Description** | **Object** | **Type** |
|---|---|---|---|
| Thumb-Tap | Tap thumb on steering wheel rim | Small | Discrete |
| Thumb-Glide | Glide thumb along steering wheel rim | Small | Continuous |
| Thumb-Long-Press | Hold thumb on steering wheel rim | Small | Continuous |
| Grab | Grab hold of steering wheel rim | Large | Continuous |
| Rim-Tap | Tap hand on steering wheel rim | Large | Discrete |

These gesture components are alternatively referred to as follows.

| **Component** | **Alternative Name** |
|---|---|
| Thumb-Tap | small-object tap |
| Thumb-Glide | small-object glide |
| Thumb-Long-Press | small-object touch-and-hold |
| Grab | large-obiect grab |
| Rim-Tap | large-object tap |

The parameters are the same for all gesture components; namely, time stamp, start angle (min_angle), end angle (max_angle), center angle (angle) and state.

The angle parameters refer to a polar angle along the steering wheel at which the object is detected. Because of the object's size, there is a first polar angle at which the object begins (start angle) and a last polar angle at which the object ends (end angle). The midpoint between the start and end angles (center angle) is used as the object's polar angle. The start and end angles are useful for determining the size of a detected object.

The state parameter takes on three values: *RECOGNIZED, UPDATED* and *ENDED.* The *ENDED* state is applied to all discrete gesture components, and also when a continuous gesture component ends. The *RECOGNIZED* and *UPDATED* states are only applied to continuous gesture components. The *RECOGNIZED* state is applied when a continuous gesture component is first detected. The *UPDATED* state is applied during the course of a continuous gesture component.

The discrete gesture components, Thumb-Tap and Rim-Tap, are emitted to the clients after they happen, and then only one message is sent for the gesture component. They are only sent with the state *ENDED.*

The continuous gesture components, Thumb-Glide, Thumb-Long-Press and Grab, are emitted to the clients intermittently from the instant that they are recognized until they end when the hand or finger leaves the rim. When they are first recognized, they are sent to the network with the state *RECOGNIZED.* With a configurable interval, the gesture component is reported to the network with new parameters and the state *UPDATED.* When the gesture component ends, the gesture component is sent with the state *ENDED.*

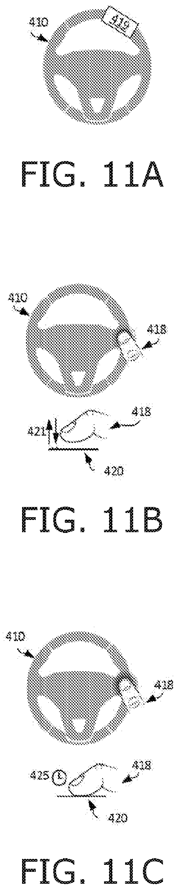

Reference is made to **FIGS. 11A** - **11E**, which are simplified illustrations of five basic gesture components used in a steering wheel user interface. **FIGS. 11A** - **11E** show the five gesture components performed by thumb **418** and hand **419** on steering wheel **410.** Some gesture components are illustrated both from above and from the side. When illustrated from the side, thumb **418** is shown interacting with steering wheel surface **420.**

**FIG. 11A** shows that a Grab gesture component is the same as a Thumb-Glide gesture component, except for two distinctions: (i) the Grab gesture is performed by a large object touching the rim, and (ii) the Grab gesture component does not have to move to be reported on the network. When the hand has been on the rim for a certain time threshold, the Grab gesture component is recognized and messages are intermittently sent to the network. **FIG. 11A** shows the Grab gesture component from above by showing hand **419** gripping steering wheel **410.**

**FIG. 11B** shows that a Thumb-Tap gesture component is generated when a small object touches, or gets very close to, the rim and is then lifted from the rim within a short period of time. This period is configurable, but typically it is 100-200 ms. **FIG. 11B** shows the Thumb-Tap gesture component from above and from the side, and illustrates the movement of thumb **418** by arrows **421.**

**FIG. 11C** shows that a Thumb-Long-Press gesture component is generated when a small object is present, and not moving, on the rim. When the small object has been present for a certain time, messages are sent intermittently to the network about the gesture component. If the object starts moving, the Thumb-Long-Press gesture component is ended and a Thumb-Glide gesture component is started instead. **FIG. 11C** shows the Thumb-Long-Press gesture component from above and from the side. Clock icon **425** indicates the time threshold required to distinguish this gesture component from a Thumb-Tap.

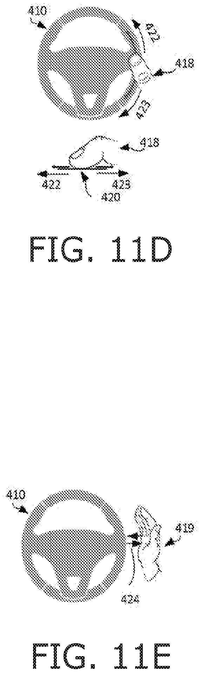

**FIG. 11D** shows that a Thumb-Glide gesture component is generated when a small object touches the rim and moves at least a certain threshold distance along the rim. That distance is configurable. When it continues to move, *UPDATE* messages are sent when the object has moved a certain distance, also configurable. **FIG. 11D** shows the Thumb-Glide gesture component from above and from the side, and illustrates the movement of thumb **418** by arrows **422** and **423.**

**FIG. 11E** shows that a Rim-Tap gesture component is the same as a Thumb-Tap, but for a large object such as a hand. **FIG. 11E** shows the Rim-Tap gesture component from the side and illustrates the movement of hand **419** by arrows **424.**

As mentioned above, gesture components are combined into compound user interface gestures. In some cases, environment conditions at the gesture location are combined with the gesture component to define a gesture. For example, a Thumb-Tap gesture performed at one end of an illuminated portion of the rim is translated into a first command, and a Thumb-Tap gesture performed at the other end of the illuminated portion of the rim is translated into a second command. The following table lists the different gestures and compound gestures in an exemplary steering wheel user interface, the gesture components that make up each gesture, additional gesture parameters, and example context and commands for each gesture.

| **Gesture** | **Gesture Components** | **Additional Parameters** | **Example Context** | **Example Command** |
|---|---|---|---|---|
| Tap inside notch | Thumb-Tap | Thumb-tap performed at top or bottom of illuminated portion of illuminated segment of steering wheel | Cruise control is active | Increase or decrease cruise control speed in 5 mph increments |
| Tap on steering wheel outer rim | Rim-Tap | | During phone call | Reactivate phone interaction, e.g., when phone call is active for set period of time. Enables hanging up the phone call with a clockwise swipe gesture |
| Single object double-tap inside notch | Two Thumb-Taps | Thumb-taps have different time stamps, similar center angles | Vehicle is in motion | Activate cruise control and illuminate location of double-tap |
| Single object double-tap on steering wheel outer rim | Two Rim-Taps | Side of steering wheel rim (left or right) at which double-tap is performed | Car is not moving, and Park Assist icon is displayed on HUD | Activate Park Assist to park on left or right side of car, based on tapped side of rim |
| Multi-touch double-tap inside notch | Two Thumb-Taps | Thumb-taps have similar time stamps, different center angles | Autonomous drive is not active | Activate autonomous drive |
| Extended touch inside notch | Thumb-Long-Press | Thumb-long-press performed at top or bottom of illuminated portion of illuminated segment of steering wheel | Cruise control is active | Increase or decrease cruise control speed in 1 mph increments |
| Grab | Grab | | Autonomous drive is active | Deactivate autonomous drive, and enter cruise control mode |
| Swipe | Thumb-Glide | clockwise/ counter-clockwise | Cruise control is active | Increase or decrease distance from forward car in cruise control mode |
| Radial swipe | Thumb-Glide | Thumb-glide data structures have similar center angles and different radial coordinates | Cruise control is active | Open cruise control menu on HUD |
| Slide | Grab | Grab data structures have different time stamps and different center angles | Portion of steering wheel is selectively illuminated | Move illumination to new hand location (follow slide movement) |
| Switch hands | Grab | Grab data structures have different time stamps and different center angles | Portion of steering wheel is selectively illuminated | Move illumination to new hand location (jump to other side of wheel) |

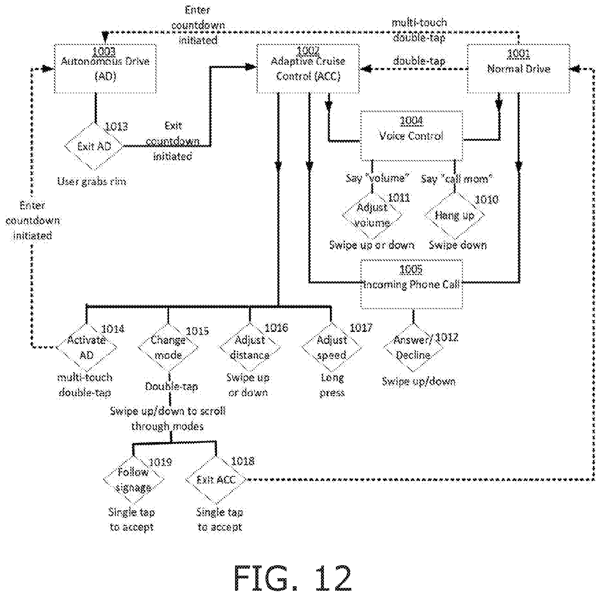

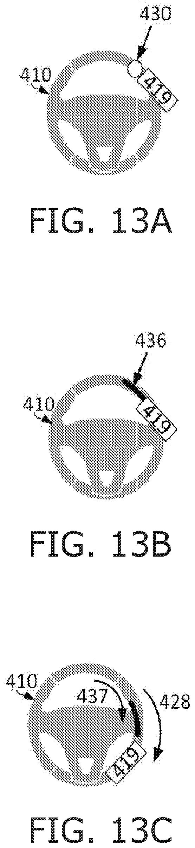

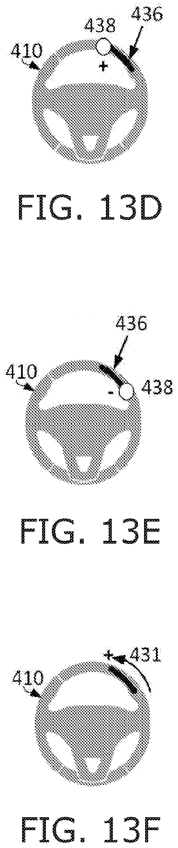

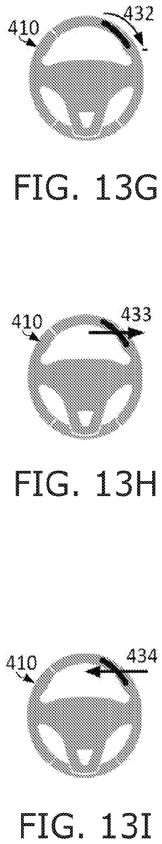

Reference is made to **FIG. 12**, which is a flowchart of an exemplary vehicle user interface. The flowchart illustrates the different application states, different commands within each state, and the gestures used to issue those commands. The details of the gestures are illustrated in **FIGS. 13** - **19**. In some embodiments a head-up display (HUD) is provided.

The flowchart of **FIG. 12** illustrates a highway scenario that includes three driving modes: Normal Drive **1001**, Adaptive Cruise Control **1002** and Autonomous Drive **1003.** In Normal Drive mode, the driver steers the vehicle and controls its speed. In Adaptive Cruise Control mode the driver steers the vehicle but the vehicle's speed, its distance from the next vehicle on the road, and other parameters are controlled by the vehicle. In Autonomous Drive mode the vehicle is driven and steered automatically without driver input.

The user enters Adaptive Cruise Control mode from Normal Drive mode by performing a double-tap gesture. The user enters Autonomous Drive mode from Normal Drive mode and from Adaptive Cruise Control mode by performing a multi-touch double-tap gesture. These gestures are described below. In order to alert the driver that Autonomous Drive mode will begin shortly, the steering wheel is illuminated with an illumination pattern that indicates a countdown until Autonomous Drive is activated.

The user exits Adaptive Cruise Control mode by performing a double-tap gesture that opens a menu on the HUD for changing the mode **1015** of cruise control. The user performs clockwise or counter-clockwise swipe gestures to scroll through the different modes on the HUD, and performs a single-tap gesture to select the displayed mode. One of the modes is Exit ACC **1018**, and selecting this mode exits Adaptive Cruise Control. Another mode configures the cruise control application to follow the road signage **1019**.

The user exits Autonomous Drive mode **1013** by grabbing the rim of the steering wheel. In order to alert the driver that Autonomous Drive mode is about to exit, the steering wheel is illuminated with an illumination pattern that indicates a countdown until Autonomous Drive is deactivated. Upon exiting Autonomous Drive mode, the vehicle enters Adaptive Cruise Control mode.

In Adaptive Cruise Control mode **1002** the user adjusts a distance **1016** between the vehicle and the vehicle directly in front of it, by performing a clockwise or counter-clockwise swipe gesture. The user adjusts the speed of the vehicle **1017** by performing either a tap gesture or an extended touch gesture. When the vehicle enters Adaptive Cruise Control mode **1002** a segment of the steering wheel is illuminated. A tap gesture or extended touch gesture at one end of the illuminated segment increases the vehicle speed, and a tap gesture or extended touch gesture at the other end of illuminated segment decreases the vehicle speed.

A voice control state **1004** can be entered from Normal Drive mode and Adaptive Cruise Control mode. In this state, the user can initiate a phone call by saying "*call*" and the name of a contact from his phone's contact list. Once the call has been connected, the user can hang up **1010** by performing a clockwise swipe gesture. The user can also adjust the volume **1011** by saying the word "*volume*" and then performing a counter-clockwise swipe gesture to raise the volume, or a clockwise swipe gesture to lower the volume.

When an incoming phone call **1005** is received, the user can answer the call **1012** by performing a counter-clockwise swipe gesture, or decline the call **1012** by performing a clockwise swipe gesture.

Reference is made to **FIGS. 13A** - **13I**, which are simplified illustrations of user interface gestures performed on a steering wheel for an adaptive cruise control function. In order to enter Adaptive Cruise Control mode from Normal Drive mode the user performs a single-object double-tap gesture. Namely, the user taps twice with his thumb on the thumb notch in the steering wheel. This gesture is shown in **FIG. 13A**, showing steering wheel **410,** hand **419** gripping steering wheel **410,** and double-tap gesture **430.** The present example enables the user to perform the double-tap gesture **430** at any location along the perimeter of steering wheel **410.**

When Adaptive Cruise Control is active the user has four options; namely, adjust cruise control speed, adjust the distance between the vehicle and the vehicle ahead, open an adaptive cruise control menu, and activate Autonomous Drive mode. As mentioned above Adaptive Cruise Control is activated when the user taps twice with his thumb in the steering wheel thumb notch. The location of these taps is subsequently illuminated to indicate to the user where to perform future gestures. This is illustrated in **FIG. 13B**, which shows illuminated segment **436** of the steering wheel **410** at the location at which double-tap **430** was performed. Thus, to increase the cruise control speed the user performs a gesture, e.g., a single-tap, above the illuminated portion. This is illustrated in **FIG. 13D**, which shows tap gesture **438** at the counter-clockwise edge of illuminated portion **436**. The "+" indicates that this gesture increases the speed of the vehicle. **FIG. 13E** shows gesture **438** performed at the clockwise end of illuminated portion **436**, and the "-" indicates that the gesture decreases the speed of the vehicle.

If the user slides his hand **419** along steering wheel **410**, the illuminated portion **436** moves with the hand so that the user's thumb is always next to the illuminated portion of the steering wheel. This is shown in **FIG. 13C**, in which hand **419** gripping steering wheel **410** slides clockwise as indicated by arrow **428**, and illuminated portion **436** also slides in the same direction as indicated by arrow **437.**

In some examples, not according to the invention, the cruise control speed is also adjusted in response to extended touch gestures above and below the illuminated portion of the steering wheel. For example, the speed is adjusted by 5 km/h in response to a tap gesture, and is adjusted by 1 km/h in response to an extended touch gesture.

In order to increase or decrease the distance between the vehicle and the vehicle in front of it on the road, the user performs clockwise and counter-clockwise swipe gestures. These are illustrated in **FIGS. 13F** and **13G****.** **FIG. 13F** shows a counter-clockwise gesture **431** to increase the distance between vehicles, and **FIG. 13G** shows a clockwise gesture **432** to decrease the distance between vehicles.

In order to change the mode of Adaptive Cruise Control the user performs a radial swipe gesture with his thumb across the width of the steering wheel thumb notch. This is illustrated in **FIGS. 13H** and **13I. FIG. 13H** shows swipe gesture **433** that moves outward across the width of illuminated portion **436**. **FIG. 13I** shows swipe gesture **434** that moves inward across the width of illuminated portion **436**. Either gesture causes the HUD to present a mode option for selection. The user performs a single-tap gesture with his thumb in the steering wheel notch to accept the displayed mode. The mode displayed in the HUD is changed in response to a swipe gesture. For example, a first mode is to follow road signage. If the user performs a single-tap when this mode is displayed on the HUD, the Follow Road Signage mode is activated. If the user swipes clockwise or counter-clockwise, a next or previous mode is displayed such as exit Adaptive Cruise Control. The user performs a single-tap to activate this mode. If no interaction from the user is received within a fixed amount of time, such as 5 seconds, then the change mode user interface is deactivated.

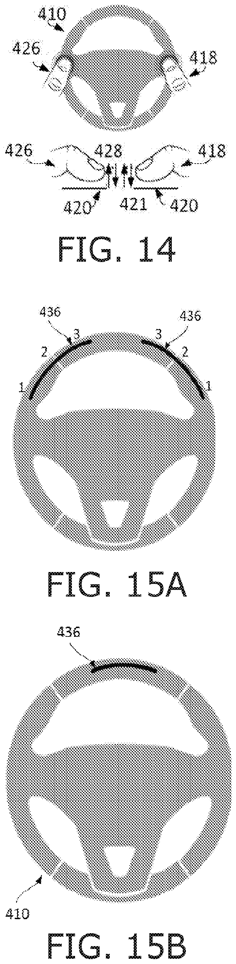

Reference is made to **FIG. 14**, which is a simplified illustration of a multi-touch double-tap gesture to activate an autonomous drive mode.

**FIG. 14** illustrates two fingers, **418** and **426**, simultaneously tapping at two locations on steering wheel **410.** The upper part of this drawing is a view from above, and the lower part of this drawing is a view from the side of each of the fingers **418** and **426.** The tap gesture is a brief down and up gesture illustrated by arrows **421** and **428** touching surface **420** of the steering wheel.

Reference is made to **FIGS. 15A** and **15B**, which are simplified illustrations of a user interface indicating activation of an autonomous drive mode. Once the user performs the multi-touch double-tap gesture of **FIG. 14****,** a series of locations on the steering wheel is sequentially illuminated over time to indicate a countdown until Autonomous Drive is activated, as shown in **FIGS. 15A** and **15B****.** For example, viewing the upright steering wheel as a clock, **FIG. 15A** shows a sequence of illuminations that begins with (i) the 2:30 and 9:30 clock positions indicated by a 1; followed by (ii) the 1:30 and 10:30 clock positions indicated by 2; followed by (iii) the 12:30 and 11:30 clock positions indicated by 3. **FIG. 15B** shows finally illuminating the 12 o'clock position to inform the user that Autonomous Drive is activated and the user can safely take his hands off the wheel.

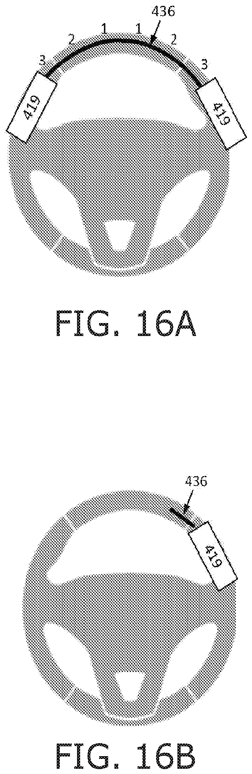

In order to exit Autonomous Drive mode and enter Adaptive Cruise Control mode, the user grabs the steering wheel. Reference is made to **FIGS. 16A** and **16B**, which are simplified illustrations of a gesture and an exemplary user interface for exiting autonomous drive mode. **FIG. 16A** shows two hands **419** gripping steering wheel **410.** A series of locations on the steering wheel is then sequentially illuminated to indicate that Autonomous Drive mode is about to be the deactivated. For example, **FIG. 16A** shows a sequence of illuminations that begins with (i) the 11:30 and 12:30 clock positions indicated by a 1; followed by (ii) the 10:30 and 1:30 clock positions indicated by 2; followed by (iii) the 9:30 and 2:30 clock positions indicated by 3. When Autonomous Drive mode is deactivated the vehicle enters Adaptive Cruise Control mode, and a portion **436** of steering wheel **410** next to one of the hands **419** gripping the steering wheel is illuminated, as shown in **FIG. 16B**, and as discussed above with reference to **FIG. 13****.**

In both Normal Drive mode and Adaptive Cruise Control mode the user can enable voice activated controls by tapping twice on the outer rim of the steering wheel. When voice-activated controls are enabled the user disables these controls by repeating the same double-tap gesture.

Two voice-activated controls are illustrated in **FIG. 12****:** placing a phone call and enabling volume adjustments. To place a phone call the user says "*call*" and the name of the person to call, e.g., "*call Mom*". In order to hang up the call the user performs a swipe gesture along the thumb notch in the steering wheel. To adjust the volume of a call or the stereo system, the user says the word "*volume*" and then adjusts the volume up or down by swiping clockwise or counter-clockwise along the thumb notch in the steering wheel.

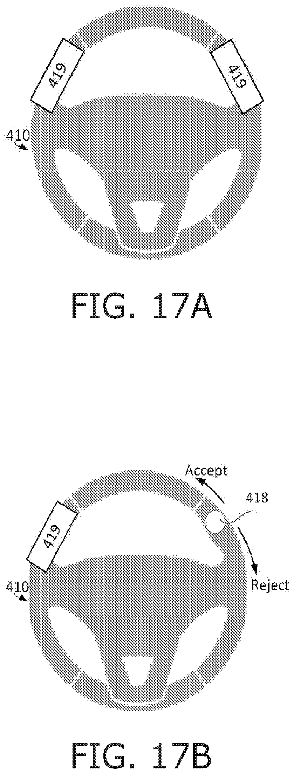

Reference is made to **FIGS. 17A** and **17B**, which are simplified illustrations showing how an incoming call is received. **FIG. 17A** shows two hands **419** gripping wheel **410,** and **FIG. 17B** shows that when an incoming call is received, the user answers or declines the call by swiping finger **418** of one hand gripping the wheel, clockwise or counter-clockwise along the thumb notch of the steering wheel, e.g., swipe counter-clockwise to accept the call and swipe clockwise to reject the call.

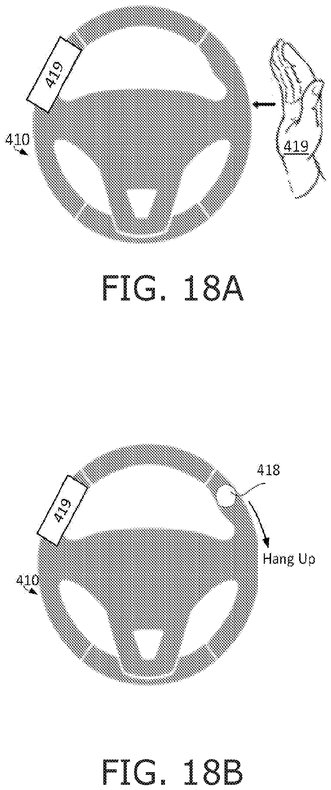

Reference is made to **FIGS. 18A** and **18B**, which are simplified illustrations showing how to hang up a call. The gesture to hang up a call is a clockwise swipe gesture. However, when a call has been active for a certain amount of time, the system ignores clockwise swipe gestures so that the user does not inadvertently hang up the call. In order to hang up the call, the user first taps the outer rim of the steering wheel, as shown by hand **419** in **FIG. 18A**, to indicate that the system should respond to the next swipe gesture, followed by a clockwise swipe gesture by finger **418** to hang up the call, as shown in **FIG. 18B****.**

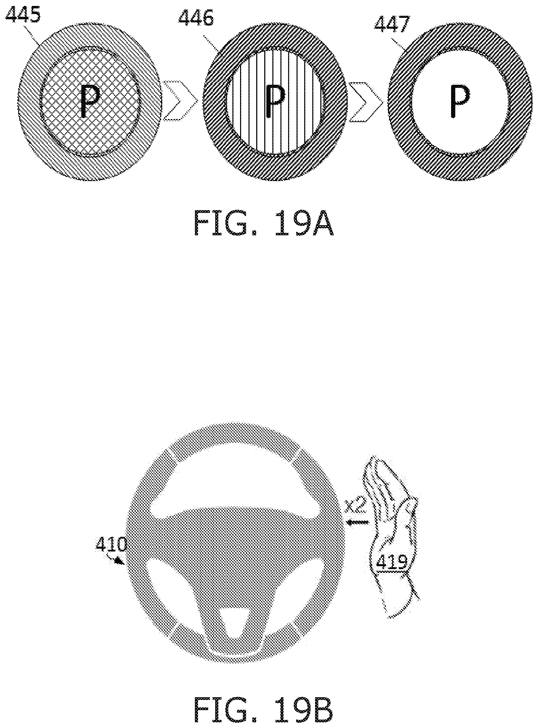

In a city scenario the user interface provides a park assist function that automatically parks the car without the user's intervention. Reference is made to **FIGS. 19A** and **19B**, which are simplified illustrations of a user interface for a park assist function. When the vehicle is moving at less than 30 km/h, the Park Assist function begins automatically scanning for available parking spaces. In addition, a faded Park Assist icon **445** appears on the HUD, as illustrated in **FIG. 19A****.** As the car further slows down, this icon becomes bolder **446**, **447** until the car has stopped moving. The HUD then presents information about available parking spots, e.g., whether the vehicle can fit into an available spot. The user performs a double-tap on the outer rim of the steering wheel, as illustrated in **FIG. 19B**, by hand **419** to begin the automated parking. To indicate to the Park Assist function that the parking space is on the left side of the car, the user performs this double-tap on the left half of the steering wheel rim. To indicate to the Park Assist function that the parking space is on the right side of the car, the user performs this double-tap on the right half of the steering wheel rim.

Embodiments known from US Publication No. 2015/123947 A1 do not form part of the invention.

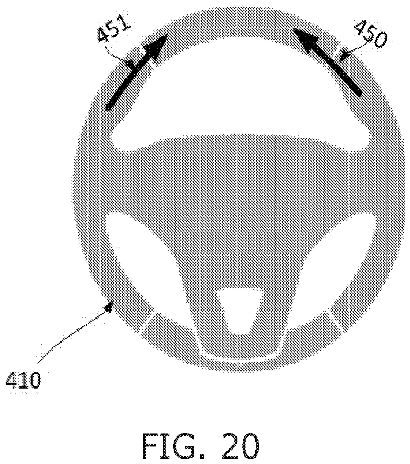

In certain examples, multiple touches on different touch-sensitive areas of the steering wheel are combined to form a compound gesture. Reference is made to **FIGS. 20** - **26**, which are simplified illustrations of multi-finger pinch and stretch gestures. **FIG. 20** shows a compound multi-finger gesture along a steering wheel rim. In **FIG. 20** two objects simultaneously move toward each other along opposing portions of steering wheel rim **410,** as indicated by movement arrows **450** and **451.** In certain examples this multi-finger gesture is interpreted as a two-finger pinch gesture on a touch screen. In certain in-vehicle entertainment and navigation systems, in response to this gesture, a displayed image is zoomed out.

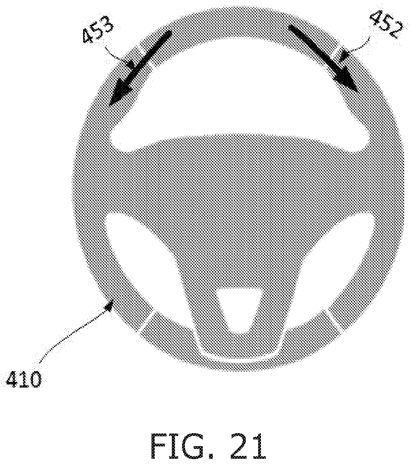

**FIG. 21** shows a second compound multi-finger gesture along a steering wheel rim. In **FIG. 21** two objects simultaneously move away from each other along opposing portions of steering wheel rim **410,** as indicated by arrows **452** and **453.** In certain examples this multi-finger gesture is interpreted as a two-finger stretch gesture on a touch screen. In certain in-vehicle entertainment and navigation systems, in response to this gesture, a displayed image is zoomed in.

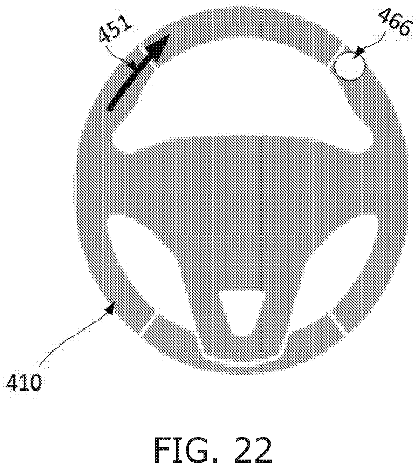

In certain examples, both objects touching the wheel need not move; the gesture is defined by the relative movement between the two objects. If one object remains in place while the other object moves, the relative movement of the two objects defines the gesture. **FIG. 22** shows a compound multi-finger gesture performed on steering wheel **410** in which element **466** is a finger that remains at one place throughout the gesture, while a second finger moves clockwise along the steering wheel rim, as indicated by arrow **451**, progressively decreasing the distance between the two fingers. This gesture is interpreted as a pinch gesture.

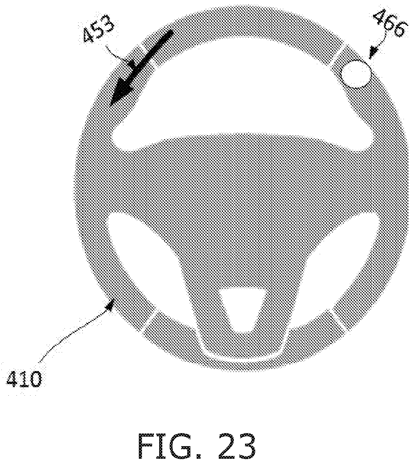

**FIG. 23** shows a compound multi-finger gesture performed on steering wheel **410** in which element **466** is a finger that remains at one place throughout the gesture, while a second finger moves counterclockwise along the steering wheel rim, as indicated by arrow **453**, progressively increasing the distance between the two fingers. This gesture is interpreted as a stretch gesture.

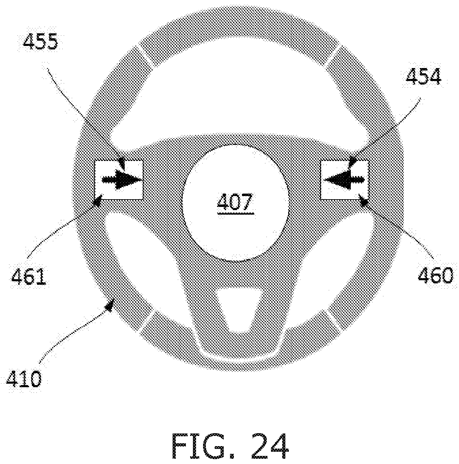

**FIG. 24** shows a compound multi-finger gesture performed on touch pads provided on spokes of a steering wheel. In **FIG. 24** two objects simultaneously move toward each other along touch pads **460** and **461** situated on opposite sides of central hub **407**, as indicated by arrows **454** and **455.** In certain examples this multi-finger gesture is interpreted as a two-finger pinch gesture on a touch screen. In certain in-vehicle entertainment and navigation systems, in response to this gesture, a displayed image is zoomed out.

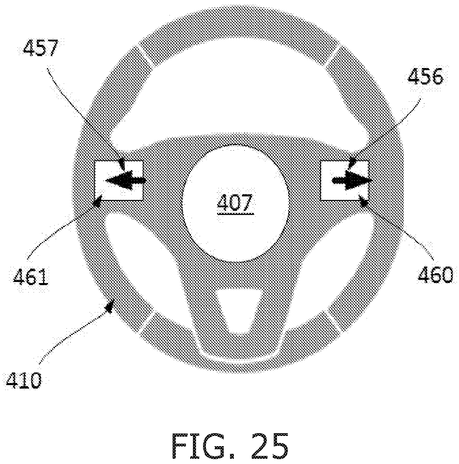

**FIG. 25** shows a compound multi-finger gesture performed on touch pads provided on spokes of a steering wheel. In **FIG. 25** two objects simultaneously move away from each other along touch pads **460** and **461** situated on opposite sides of central hub **407**, as indicated by arrows **456** and **457.** In certain examples this multi-finger gesture is interpreted as a two-finger stretch gesture on a touch screen. In certain in-vehicle entertainment and navigation systems, in response to this gesture, a displayed image is zoomed in.

In examples, a user performs gestures on two separate areas on or around the steering wheel, and those gestures are interpreted as a single, multi-finger gesture. The multi-finger gesture is defined by the movement of the fingers in relation to each other, as in pinch gestures and rotation gestures.

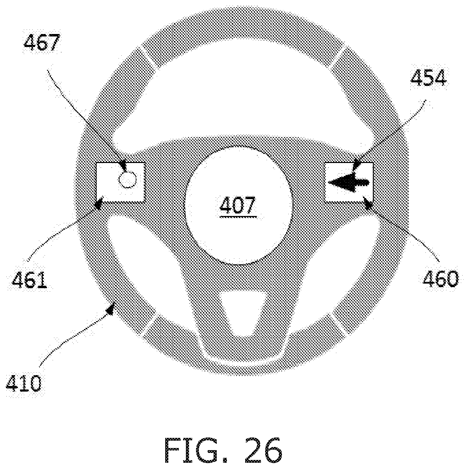

**FIG. 26** shows a compound multi-finger gesture performed on touch pads **460** and **461** on steering wheel **410.** Element **467** indicates a finger that remains at one place on touch pad **461** throughout the gesture, while a second finger moves **454** across touch pad **460** toward center **407** of the wheel, progressively decreasing the distance between the two fingers. This gesture is interpreted as a pinch gesture.

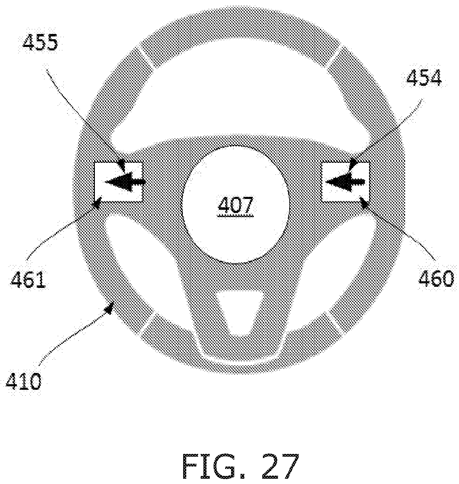

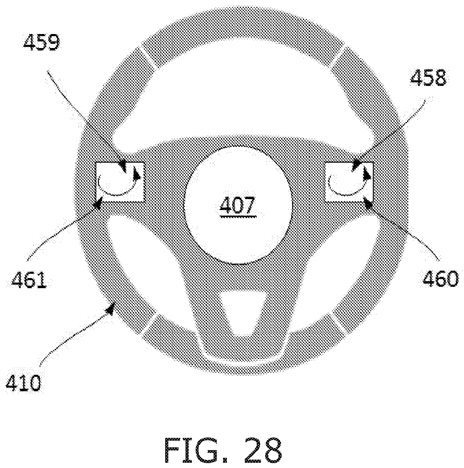

Reference is made to **FIGS. 27** and **28**, which are simplified illustrations of multi-finger gestures. **FIG. 27** shows a compound multi-finger gesture performed on touch pads **460** and **461** on steering wheel **410.** In this case both fingers move in the same direction on their respective touch pads, as indicated by arrows **454** and **455.** This gesture is interpreted as a panning gesture, in response to which the user interface pans an image.

**FIG. 28** shows a compound multi-finger gesture performed on touch pads **460** and **461** on steering wheel **410.** In this case both fingers move in tandem tracing a curve on their respective touch pads, as indicated by arrows **458** and **459.** This gesture is interpreted as a panning gesture, in response to which the user interface pans an image. The pan follows the direction of the finger movement, and is therefore not in a straight line.

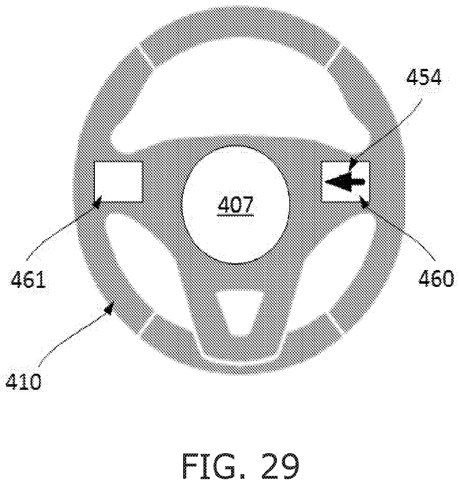

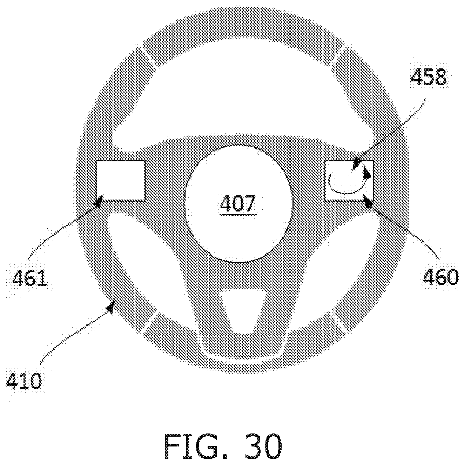

Reference is made to **FIGS. 29** and **30**, which are simplified illustrations of one-finger gestures. **FIGS. 29** and **30** show one-finger gestures performed on touch pad **460** on steering wheel **410**, as indicated by respective arrows **454** and **458.** Because only one finger is used, these gestures are interpreted differently than the multi-finger gestures discussed hereinabove. In some cases these one-finger gestures are interpreted as panning gestures, whereby the user interface pans the image at a different speed than when two-finger panning gestures are performed. In other cases the user interface behavior in response to these gestures is the same as to the gestures shown in **FIGS. 27** and **28** in which two fingers move across different surfaces in the same direction.

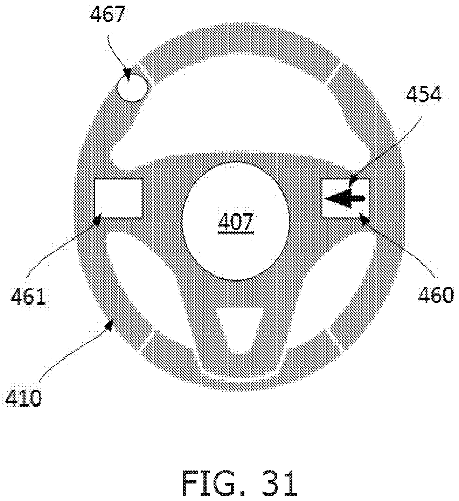

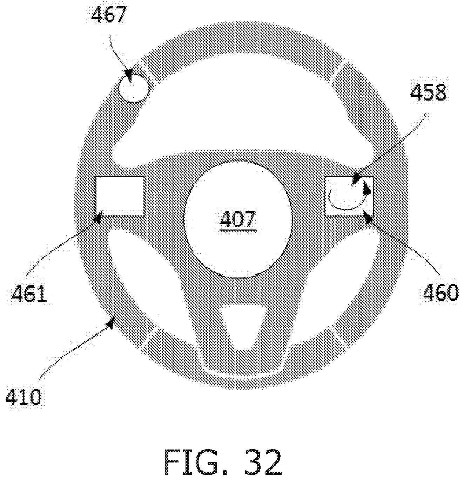

Reference is made to **FIGS. 31** - **34**, which are simplified illustrations of multi-finger gestures. **FIGS. 31** and **32** show compound gestures performed on touch pad **460** and on the rim of steering wheel **410.** In **FIGS. 31** and **32** a finger is stationary on the steering wheel rim, as indicated by element **467**, while another finger performs a moving gesture on touch pad **460**, as indicated by arrows **454** and **458.** As explained hereinabove, the compound gesture elicits a different response from the user interface than each of the individual gestures that make up the compound gesture. Moreover, in certain cases, at least one of the compound gesture's parameters is how the two or more objects performing the gesture relate to each other. For example, whether the distance between the objects is increasing, decreasing, or remains the same, i.e., the objects move in tandem with each other.

In some cases, a compound gesture represents a combination of two or more known multi-touch gestures and the steering wheel user interface reacts by executing the multiple corresponding commands simultaneously.

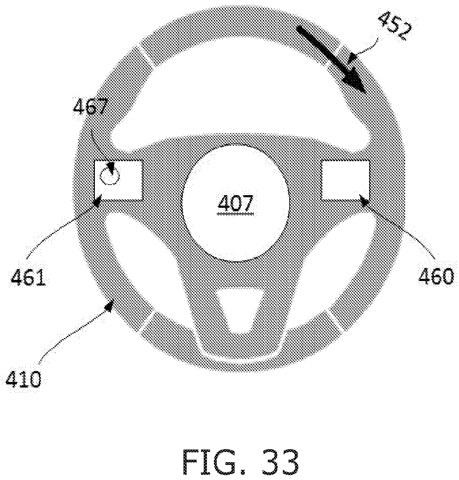

**FIG. 33** shows a compound gesture performed on touch pad **461** and on the rim of steering wheel **410.** In **FIG. 33** a finger is stationary on touch pad **461**, as indicated by element **467**, while another finger performs moving gesture **452** on the steering wheel rim. In these examples, the finger performing a gesture on the rim can be replaced by a hand. In certain examples, only finger gestures on the steering wheel rim are combined with another gesture into a compound gesture, whereas a gesture performed by a hand is not combined with another gesture into a compound gesture. Fingers and hands are distinguished from each other by their sizes as determined by the number of emitter-detector pairs that detect them.

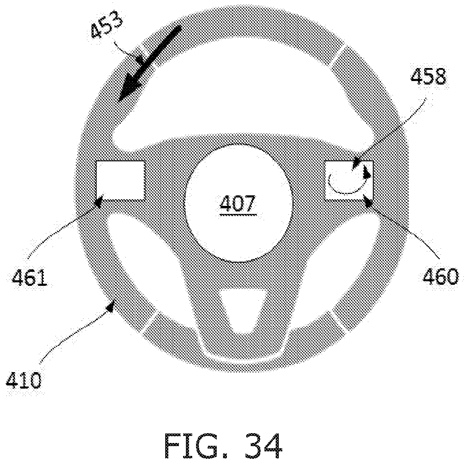

**FIG. 34** shows a compound gesture performed on touch pad **460** and on the rim of steering wheel **410.** In **FIG. 34** a finger is performing curved sweep gesture **458** on touch pad **460** while another finger performs counterclockwise sweep gesture **453** on the steering wheel rim.

Examples disclosed herein verify that the driver is gripping a steering wheel. Simply detecting that an object is gripping the steering wheel is not sufficient to determine that a driver is gripping the wheel. For example, such detection cannot distinguish a hand from an inanimate object wrapped around a portion of the steering wheel. Examples use the light-based proximity sensors in the steering wheel to distinguish between a human hand gripping the wheel and an inanimate object gripping the wheel by detecting two distinguishing features of a human hand: micro-movements and blood flow.

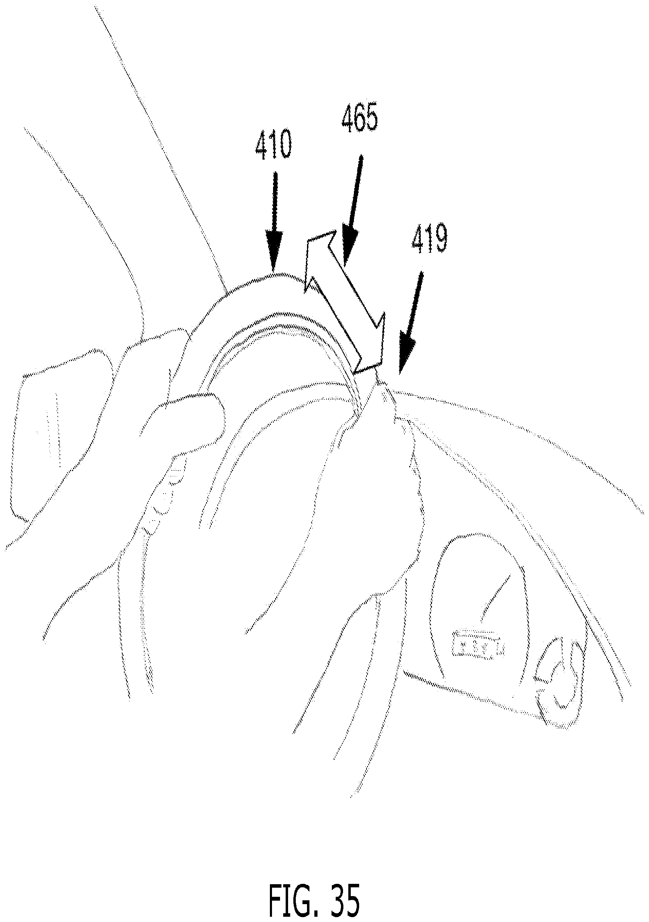

Reference is made to **FIG. 35**, which is a simplified illustration of micro-movements detected on a steering wheel. Micro-movements are very small movements of the hand gripping the wheel. **FIG. 35** shows hand **419** gripping wheel **410.** Arrow **465** illustrates the direction of micro-movements detected by the steering wheel sensor described hereinabove in relation to **FIGS. 2 - 7****.** These movements are miniscule, not as large as arrow **465**, but they cause the steering wheel sensor detections at the edges of the hand to fluctuate in response to these movements. In some examples, when the driver performs large movements of his hands on the wheel, e.g., when changing his grip or when turning the wheel, these detected large movements also serve to indicate that the driver is in control of the wheel. In these examples, micro-movement detection is only required in the absence of large movements.

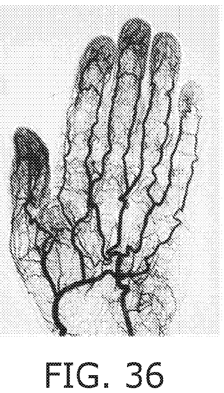

Reference is made to **FIG. 36**, which is a simplified illustration of blood flow through a hand, the blood flow being detected by a reflectance-based sensor. Blood flowing through hand **419** gripping wheel **410** in **FIG. 35** causes fluctuations in the steering wheel sensor detections when the skin of hand **419** is exposed to the thumb-receiving notch in steering wheel **410**, based on a technique used for non-invasively monitoring oxygen saturation and pulse rate known as "reflectance-based pulse oximetry". In reflectance-based pulse oximetry, incident light is passed through the skin and is reflected off the subcutaneous tissue and bone. In a steering wheel mounted proximity sensor, the infrared or near infrared light beams from the proximity sensor emitters pass through the skin of the driver's hand gripping the wheel and are reflected off the subcutaneous tissue and bone back to the proximity sensor light detectors. In some cases, a red light is used instead of infrared or near infrared light. When the driver moves his hand on the wheel, the reflectance-based pulse oximetry signals may be unstable due to user movement. In some cases, this is compensated for by signal processing by the proximity sensor controller, or by post-processing by the host processor, based on tracking the movement. In other cases, blood flow is identified only when the hand gripping the wheel is not moving. When the hand gripping the wheel is moving, the tracked movement of the hand indicates that it is a human hand gripping the wheel, and blood flow is not identified.

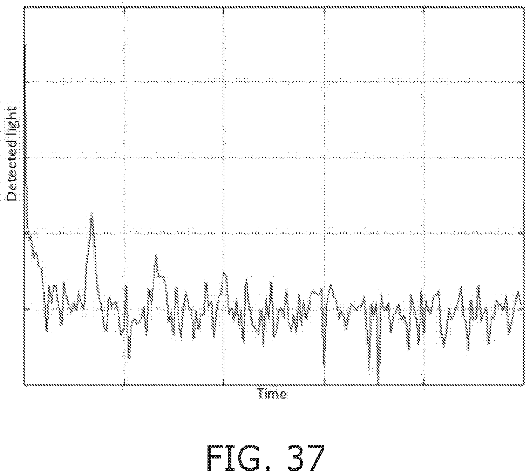

Reference is made to **FIGS. 37** - **44**, which are graphs of filtered detections of blood flow inside a hand gripping a steering wheel equipped with reflectance-based sensors. **FIG. 37** is a graph of filtered detections of a hand by the steering-wheel mounted optical proximity sensor described hereinabove using a transmit current of 150 mA to activate the emitters. The detection signal was filtered with the Fourier transform and the graph shows the expected modulation in the reflected light. In some examples, an automatic gain control loop is used for the pulse sensing channel, as saturating the receiver would block the modulation.

Since the purpose of this detection is only to distinguish a human hand from an inanimate object, it is not necessary to detect the pulse accurately, and detection of the fundamental frequency in the detections is sufficient. In certain examples, a pitch detection algorithm (PDA) is used to estimate the pitch, or fundamental frequency of the detection signal. The "harmonic product spectrum" is a pitch detection algorithm used in certain examples to identify the fundamental frequency in the detections.

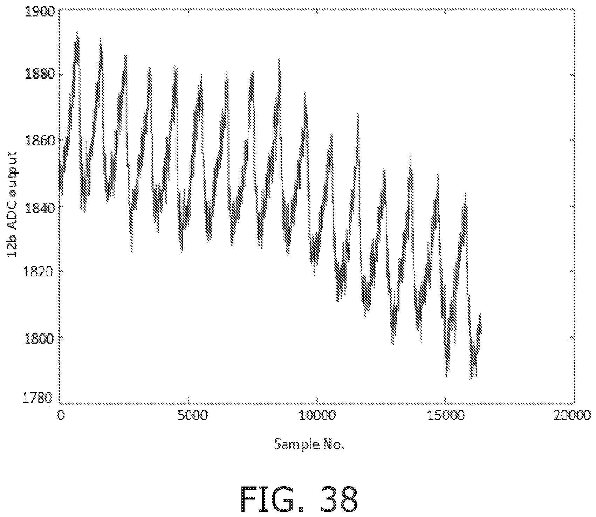

**FIG. 38** is a graph of filtered detections of a hand by the steering-wheel mounted optical proximity sensor described hereinabove. The detection signal was filtered in the time domain using a 16-point averaging filter. The graph in **FIG. 38** was generated by sampling only one channel detecting the hand, at a 1 kHz sample rate. In other examples, averaging is additionally or alternatively performed on neighboring channels within a scan sequence.

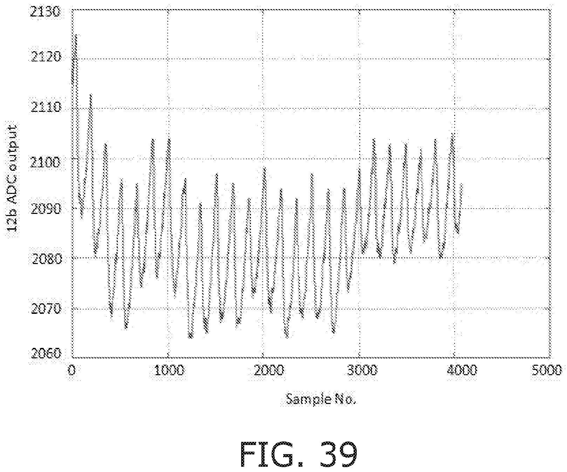

**FIG. 39** is a graph of filtered detections of a hand by the steering-wheel mounted optical proximity sensor described hereinabove. The detection signal was filtered twice using a 16-point averaging filter, i.e., the output of a 16-point averaging filter is filtered again with a 16-point averaging filter. The signal in **FIG. 39** is cleaner than that of **FIG. 38****.**

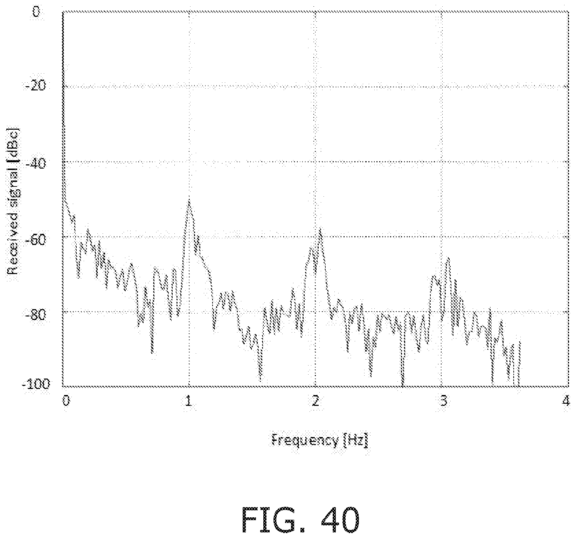

**FIG. 40** is a graph of detections of a hand by the steering-wheel mounted optical proximity sensor described hereinabove, using infrared light emitters at a rate of 74Hz, and an over-sampling ratio of 16.

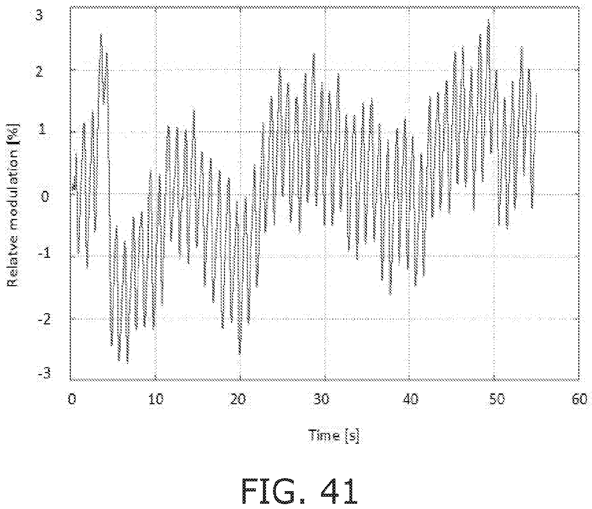

**FIG. 41** is a graph of detections of a hand by the steering-wheel mounted optical proximity sensor described hereinabove, using infrared light emitters at a rate of 74 Hz, an over-sampling ratio of 16, and a 16-tap finite impulse response (FIR) filter.

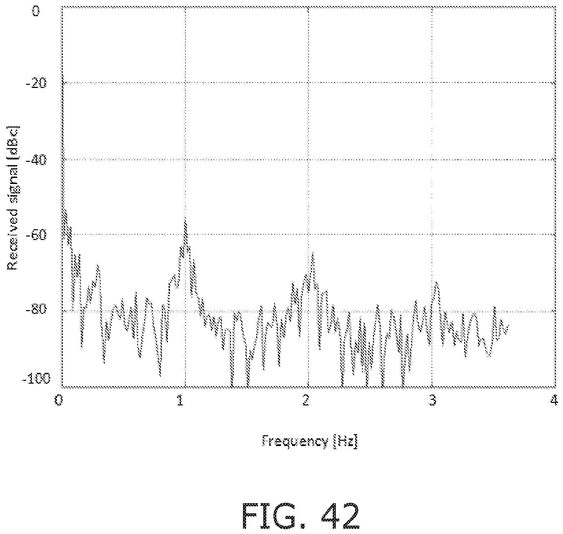

**FIG. 42** is a graph of detections of a hand by the steering-wheel mounted optical proximity sensor described hereinabove, using red light emitters at a rate of 74 Hz and an over-sampling ratio of 16.

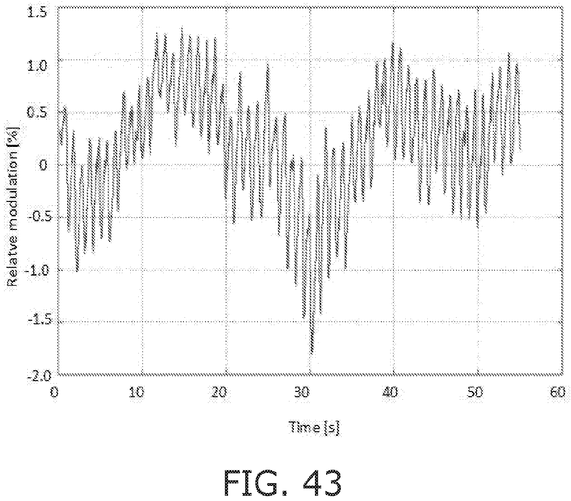

**FIG. 43** is a graph of detections of a hand by the steering-wheel mounted optical proximity sensor described hereinabove, using red light emitters at a rate of 74 Hz, an over-sampling ratio of 16, and a 16-tap FIR filter. **FIGS. 40** - **43** show that although the visible red light provides a stronger overall signal, it provides a weaker modulation than IR light. Therefore, IR light is better than visible light for detecting blood flow.

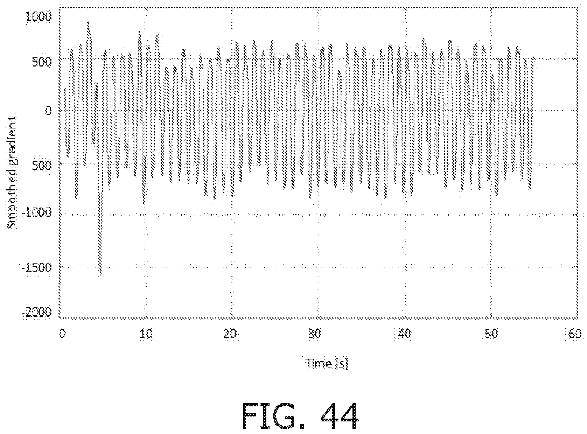

**FIG. 44** is a graph of detections of a hand by the steering-wheel mounted optical proximity sensor described hereinabove, in which the time domain signal was high-pass filtered to reject low frequency variation and then filtered again with a 32-tap FIR to smooth the waveform.

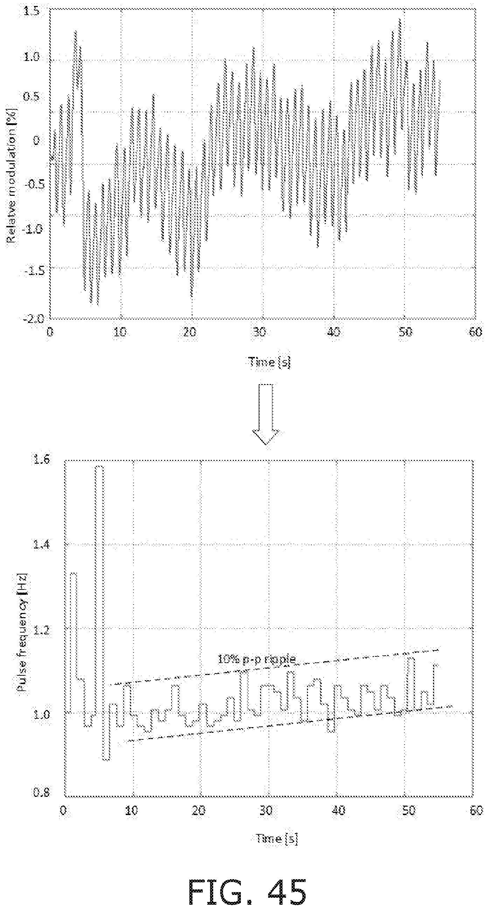

Reference is made to **FIG. 45**, which is a pair of graphs, namely, a top graph of detections of blood flow inside a hand gripping a steering wheel, and a bottom graph of an estimated period in the detections of the top graph. **FIG. 45** shows how to estimate a period in the signal of **FIG. 41**, reproduced at the top of **FIG. 45**, by calculating the number of samples between falling zero crossings. The estimated period is shown in the graph at the bottom of **FIG. 45****.** The low pass variation in the signal of **FIG. 41** is attenuated, but sharp changes affect the result. The remaining ripple on the estimated pulse frequency in the graph at the bottom of **FIG. 45** is too high.

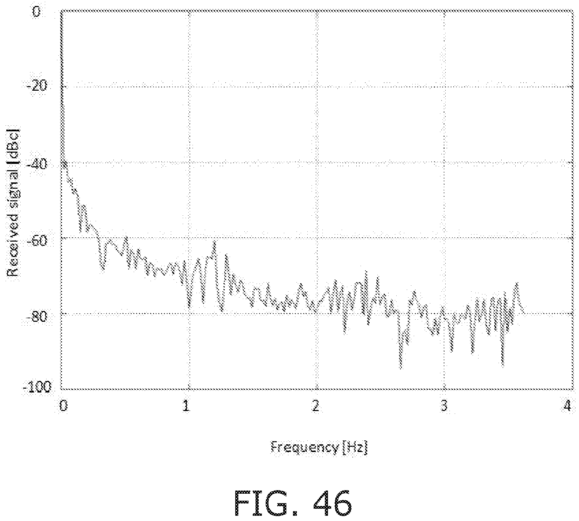

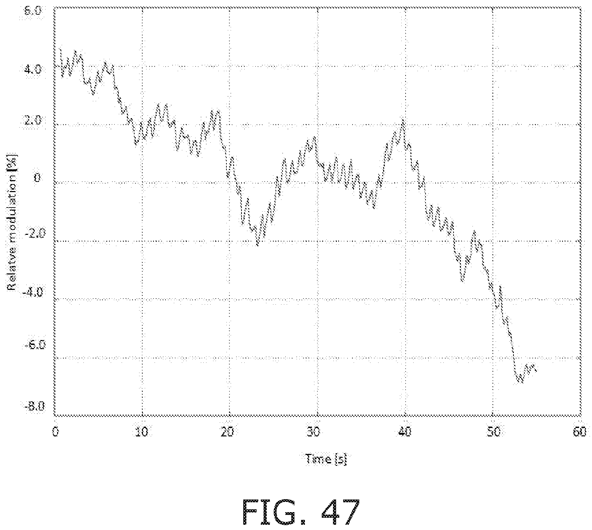

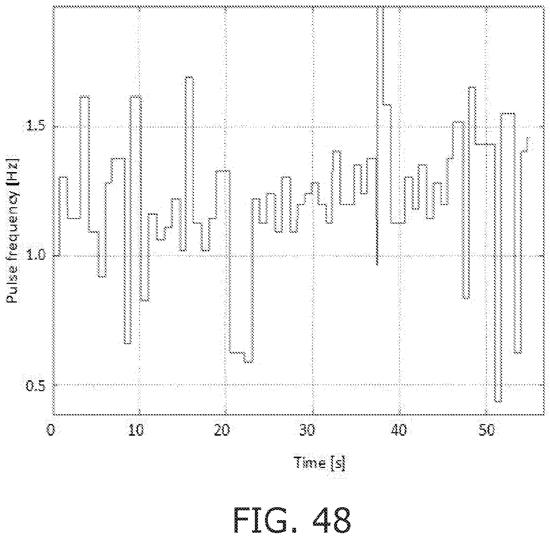

Reference is made to **FIG. 46**, which is a graph illustrating the spectrum of a pulse-detection signal. Reference is made to **FIG. 47**, which is a graph illustrating modulation of a pulse-detection signal over time. Reference is made to **FIG. 48**, which is a graph illustrating an estimated frequency of a pulse-detection signal. In examples, this pulse detection signal is used to calculate a pulse rate of the driver.

The standards organization SAE International has released a Recommended Practice document, Standard No. J3016_201609 entitled *Taxonomy and Definitions for Terms Related to Driving Automation Systems for On-Road Motor Vehicles*, that provides a taxonomy for motor vehicle driving automation systems that perform part or all of the dynamic driving task (DDT) on a sustained basis and that range in level from no driving automation (level 0) to full driving automation (level 5).

In general, SAE J3016 levels and definitions include:
- Level 0 - No Automation: The full-time performance by the human driver of all aspects of the dynamic driving task, even when enhanced by warning or intervention systems
- Level 1 - Driver Assistance: The driving mode-specific execution by a driver assistance system of either steering or acceleration/deceleration using information about the driving environment and with the expectation that the human driver performs all remaining aspects of the dynamic driving task
- Level 2 - Partial Automation: The driving mode-specific execution by one or more driver assistance systems of both steering and acceleration/deceleration using information about the driving environment and with the expectation that the human driver performs all remaining aspects of the dynamic driving task
- Level 3 - Conditional Automation: The driving mode-specific performance by an Automated Driving System of all aspects of the dynamic driving task with the expectation that the human driver will respond appropriately to a request to intervene
- Level 4 - High Automation: The driving mode-specific performance by an Automated Driving System of all aspects of the dynamic driving task, even if a human driver does not respond appropriately to a request to intervene
- Level 5 - Full Automation: The full-time performance by an Automated Driving System of all aspects of the dynamic driving task under all roadway and environmental conditions that can be managed by a human driver

Particularly in Level 3, Conditional Automation, the Automated Driving System operates in accordance with the assumption that the human driver will respond appropriately to a request to intervene. In order to ensure that the driver will intervene as expected, the present invention provides systems that measure a degree of driver preparedness to intervene.

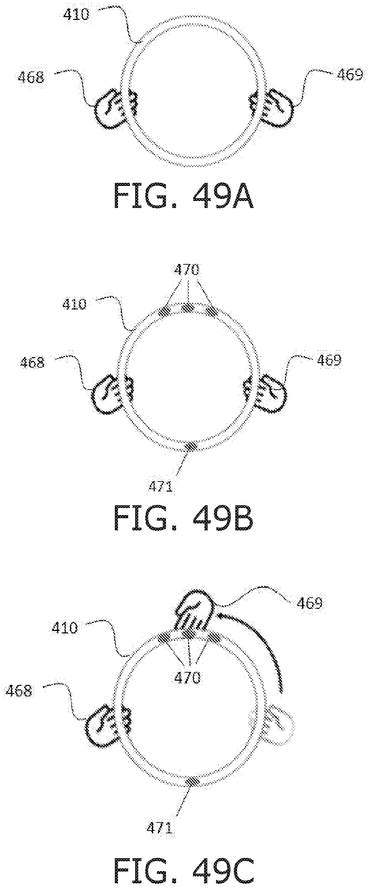

Reference is made to **FIGS. 49A** - **49C**, which are simplified illustrations of a system for measuring driver alertness, in accordance with an embodiment of the present invention. **FIG. 49A** shows steering wheel **410** provided with sensors that detect the position of hands **468** and **469** gripping the wheel. **FIG. 49B** shows that steering wheel **410** also includes illuminators, such as illuminators **470** and **471** along the perimeter of steering wheel **410**. Based on the current location of the driver's hands on steering wheel **410**, the system illuminates one or more locations separate from the driver's hands and prompts the driver to touch the illuminated portions. The prompt is, for example, an audio or visual prompt. **FIG. 49C** shows that a degree of driver preparedness is derived based on the amount of time it takes the driver to touch the illuminated portions of steering wheel **410.** The degree of driver preparedness is also derived based on how close the driver's touch is to the illumination. In some embodiments of the invention, the illumination is generated at a midpoint between the driver's hands on steering wheel **410**, and the system determines the driver's handedness based on which hand was used to touch the illumination. In some embodiments of the invention, when the sensors in steering wheel **410** detect that the driver is not currently gripping steering wheel **410**, the system prompts the driver to grip steering wheel **410** prior to illuminating portions of steering wheel **410**.

In another example, a system for measuring a degree of driver preparedness uses a touch screen in the dashboard or elsewhere in the vehicle, instead of steering wheel **410**, to measure reaction time. In this case, a location on the touch screen is indicated for the driver to touch and the time it takes the driver to touch the screen location indicates the degree of driver preparedness.

In some embodiments, the system for measuring a degree of driver preparedness is activated when the Automated Driving System predicts a likelihood that driver intervention will be required soon. This typically occurs when driving conditions change; e.g., the car navigation system instructs the car to exit the highway, it has begun to rain, or the sun is setting and it is getting dark.

In some cases, the Automated Driving System requires a minimum degree of driver preparedness to proceed. If driver preparedness is below this threshold, the Automated Driving System reacts defensively, e.g., by increasing the distance to other vehicles on the road, by reducing vehicle speed, or by navigating the vehicle to a location where it parks the car. The Automated Driving System then resumes its previous course when driver preparedness increases. Similarly, the Automated Driving System uses the degree of driver preparedness to adapt driving parameters to the driver's abilities. E.g., the Automated Driving System drives defensively for a driver whose preparedness scores are consistently low, e.g., by increasing the distance to other vehicles on the road and reducing vehicle speed. Sometimes, a low reaction time is due to the driver not noticing the illumination. Therefore, in some cases, the illumination color or intensity is modified to catch the driver's attention. Blinking the illumination is also useful for catching the driver's attention.

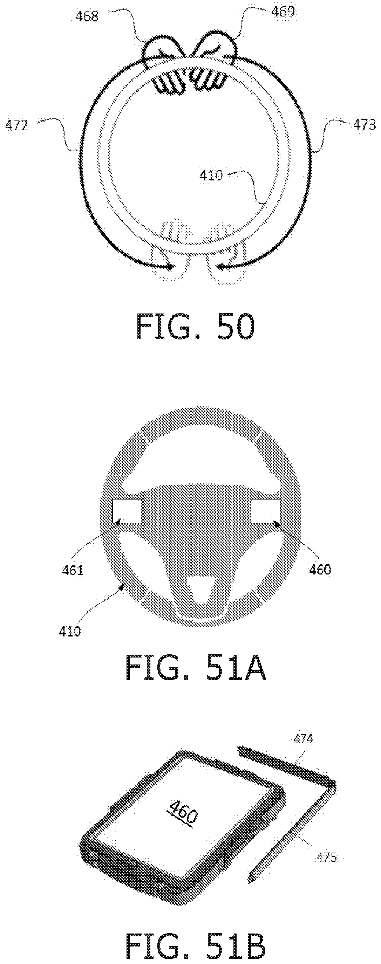

Reference is made to **FIG. 50**, which is a simplified illustration of a gesture for starting a vehicle. **FIG. 50** shows a gesture for starting a vehicle engine for vehicles that include an array of sensors along the perimeter of the steering wheel grip. The gesture of **FIG. 50** involves the user's hands **468**, **469** sliding **472**, **473** along the grip around the steering wheel **410**. In some cases, the gesture is performed symmetrically by both hands simultaneously, each hand performing an extended slide gesture along a different half of the steering wheel grip. For example, the left hand slides downward along the left half of the grip, while the right hand slides downward along the right half of the grip. In other cases, this gesture is used for activating functions other than starting the engine.

The gesture of **FIG. 50** has several benefits. By performing the gesture across the entire steering wheel grip, all sensors in the grip are tested, ensuring that all sensors are working properly each time the car is started. The vehicle generates an error state when not all locations along the length of the grip detect touch.

Furthermore, since the sensor is reflection-based, when the driver wears gloves the sensor detects different amounts of light than when the driver is not wearing gloves. Also, even without gloves, different skin tones reflect different amounts of light. Requiring the driver to perform this extended slide gesture when starting the vehicle, provides an opportunity to calibrate the sensors for the current driver's hands when this extended gesture is performed. In addition, the extended slide gesture wipes away dirt and dust from the grip that would interfere with the steering wheel touch sensor.

Reference is made to **FIGS. 51A**, **51B**, **52** and **53**, which are simplified illustrations of systems with duplicated touch sensors. **FIGS. 51A** and **51B** show a first embodiment of a duplicated sensor system for touch pad controls. **FIG. 51A** shows touch pads **460** and **461** in steering wheel **410**. Use of these pads is discussed hereinabove with reference to **FIGS. 24** - **34****.** **FIG. 51B** shows two sensors **474** and **475** placed along different edges of pad **460**, each sensor **474** and **475** providing independent touch and gesture detection on pad **460**. Resistive and capacitive sensors require a film or layer on the touch surface, which precludes providing two independent resistive or capacitive sensors on the same area or on a steering wheel grip. In contrast, reflection-based sensors are useful for providing multiple independent sensors for the same area. Thus, for example, a touch pad or steering wheel is covered with a capacitive sensor, and in addition, a reflectance based sensor provides a second sensor thereon. In other cases, two or more reflectance-based sensors provide duplicate sensing to the same area by placing different sensors along different edges of the area, enabling up to four independent reflectance-based sensors detecting touch and gestures on any rectangular area.

Alternatively the different reflectance-based sensors are stacked one above the other on a common edge of the detection area. In this case, the different sensors detect objects in adjacent detection planes, but all sensors will detect gestures and touches on the touch pad.

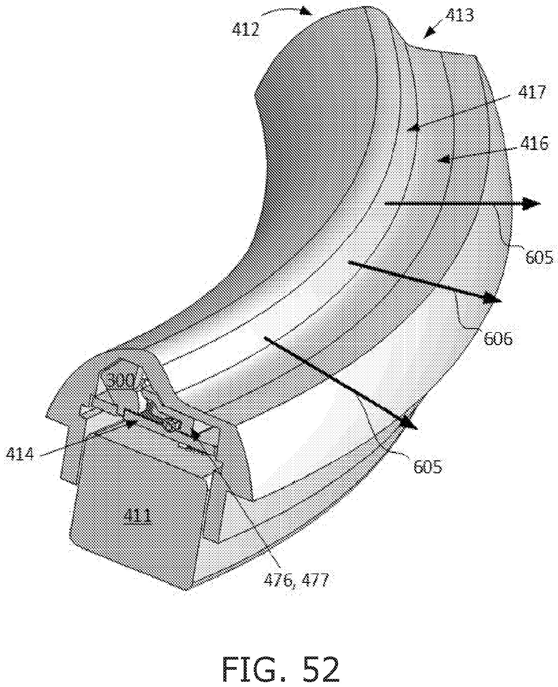

**FIG. 52** shows a second duplicated sensor system. In this case the emitters and detectors of two reflectance-based sensors are interleaved. **FIG. 52** illustrates this duplicated sensor system in a section of a steering wheel grip. **FIG. 52** shows emitter beams **605** from a first of the two sensors, and emitter beam **606** from the second of the two sensors. In this configuration of interleaved sensor components, both sensors detect objects in the same plane. Each sensor is controlled by a respective one of CPUs **476, 477.** Steering wheel frame **411,** top cover **412,** thumb-notch **413**, PCB **414**, and transparent cover sections **416** and **417** are described hereinabove with reference to **FIGS.** 2 - 9.

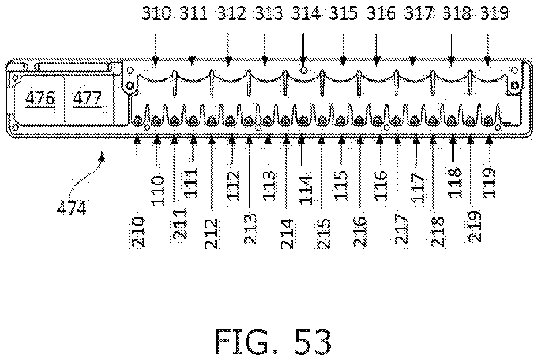

**FIG. 53** shows interleaved emitters and detectors of two sensors in a duplicated sensor system. **FIG. 53** illustrates a duplicated sensor system in a one-dimensional sensor array **474.** Each sensor is controlled by a respective one of processors **476**, **477.** Every other emitter, together with its right-neighbor detector belongs to a first of the sensors and the remaining emitters and detectors belong to the other sensor; i.e., even-numbered emitters and detectors **110**, **210**, **112**, **212**, ..., **118**, **218**, are controlled by CPU **476** and odd-numbered emitters and detectors **111**, **211**, **113**, **213**, ..., **119**, **219**, are controlled by CPU **477.** Lenses **310** - **319** direct light from the emitters out of sensor array **474** and direct object reflections onto the detectors, as explained hereinabove with reference to **FIG. 8****.** In other arrangements place two detectors between every two emitters, whereby every other emitter, together with one of its right-neighbor detectors and one of its left-neighbor detectors, belongs to a first of the sensors and the remaining emitters and detectors belong to the other sensor.

In the foregoing specification, the invention has been described with reference to specific exemplary embodiments thereof. It will, however, be evident that various modifications and changes may be made to the specific exemplary embodiments without departing from the invention. In particular, sensors other than optical sensors may be used to detect the user interface gestures, inter alia capacitive sensors disposed along the circumference of the steering wheel, and cameras that capture images of the steering wheel. Accordingly, the specification and drawings are to be regarded in an illustrative rather than a restrictive sense. The scope of protection is defined by the claims.

## Claims

1. A vehicle comprising:
an advanced driver-assistance system (ADAS) that selectively performs, and selectively stops performing, automated vehicle navigation operations;
a steering wheel (410) with which a human driver steers the vehicle when the automated vehicle navigation operations are not performed;
a sensor (414, 100, 200, 300, 440) operable to detect a driver's hand at a plurality of locations on said steering wheel;
a plurality of visible-light emitters (106 - 109) operable to illuminate the plurality of locations on said steering wheel; and
a processor (441 - 443), coupled with said ADAS, with said sensor and with said visible-light emitters, performing the following functions while said ADAS is performing the automated vehicle navigation operations:
(i) an activating function that activates at least one of said emitters to illuminate a location on said steering wheel,
(ii) a detecting function that detects, via said sensor, when the driver touches the illuminated location, and
(iii) a calculating function that calculates a degree of driver preparedness for taking control of steering the vehicle when said ADAS stops performing the automated vehicle navigation operations, based on at least one of time elapsed between the activating function and the detecting function, and proximity of the driver touch to the illuminated location.

2. The vehicle of claim **1**, further comprising an audio system, wherein the activation function causes the audio system to provide an audio prompt to the driver to touch the illuminated location.

3. The vehicle of claim **1**, wherein said processor (441 - 443) detects, via said sensor, an initial location of the driver's hand on said steering wheel prior to performing the activating function, and selects a location on said steering wheel to illuminate based on the detected initial location.

4. The vehicle of claim **1**, wherein said processor (441 - 443) detects, via said sensor, initial locations of the driver's two hands on said steering wheel prior to performing the activating function, and further identifies the driver's handedness based on which hand touches the illuminated location.

5. The vehicle of claim **1**, wherein said ADAS configures a parameter of the automated vehicle navigation operations in response to the degree of driver preparedness calculated by said processor.

6. The vehicle of claim **5**, wherein the parameter is a distance from a vehicle ahead.

7. The vehicle of claim **5**, wherein the parameter is a vehicle speed.

8. The vehicle of claim **1**, wherein said ADAS navigates the vehicle to a parking location in response to a low degree of driver preparedness calculated by said processor.

9. The vehicle of claim **1**, wherein said processor (441 - 443) configures the activating function based on a prior calculated degree of driver preparedness.

10. The vehicle of claim **9**, wherein the activating function selects an illumination color based on the prior calculated degree of driver preparedness.

11. The vehicle of claim **1**, wherein said ADAS instructs said processor (441 - 443) to perform the activating, detecting and calculating functions, based on an anticipated change in driving conditions.

12. The vehicle of claim **11**, wherein the anticipated change in driving conditions is an automated navigation instruction to exit a highway.

## Patentansprüche

1. Fahrzeug, umfassend:
ein fortschrittliches Fahrerassistenzsystem (ADAS), das die automatische Fahrzeugnavigation selektiv durchführt und selektiv beendet;
ein Lenkrad (410), mit dem ein menschlicher Fahrer das Fahrzeug lenkt, wenn die automatischen Fahrzeugnavigationsvorgänge nicht durchgeführt werden;
einen Sensor (414, 100, 200, 300, 440), der die Hand des Fahrers an mehreren Stellen des Lenkrads erkennen kann;
eine Vielzahl von Sendern für sichtbares Licht (106 - 109), die so betrieben werden können, dass sie die Vielzahl von Stellen auf dem Lenkrad beleuchten; und
einen Prozessor (441 - 443), der mit dem ADAS, mit dem Sensor und mit den Sendern für sichtbares Licht gekoppelt ist und die folgenden Funktionen ausführt, während das ADAS die automatischen Fahrzeugnavigationsvorgänge durchführt:
(i) eine Aktivierungsfunktion, die mindestens einen der Sender aktiviert, um eine Stelle des Lenkrads zu beleuchten,
(ii) eine Erkennungsfunktion, die über den Sensor erkennt, wenn der Fahrer die beleuchtete Stelle berührt, und
(iii) eine Berechnungsfunktion, die einen Grad der Bereitschaft des Fahrers berechnet, die Kontrolle über die Lenkung des Fahrzeugs zu übernehmen, wenn das ADAS aufhört, die automatisierten Fahrzeugnavigationsvorgänge durchzuführen, basierend auf mindestens einer der folgenden Größen: Zeit, die zwischen der Aktivierungsfunktion und der Erkennungsfunktion verstrichen ist, und Nähe der Berührung des Fahrers zu dem beleuchteten Ort.

2. Fahrzeug nach Anspruch **1**, das ferner ein Audiosystem umfasst, wobei die Aktivierungsfunktion das Audiosystem veranlasst, dem Fahrer eine akustische Aufforderung zu geben, die beleuchtete Stelle zu berühren.

3. Fahrzeug nach Anspruch **1**, wobei der Prozessor (441 - 443) über den Sensor eine anfängliche Position der Hand des Fahrers auf dem Lenkrad erkennt, bevor die Aktivierungsfunktion ausgeführt wird, und eine zu beleuchtende Stelle auf dem Lenkrad basierend auf der erkannten anfänglichen Position auswählt.

4. Fahrzeug nach Anspruch **1**, wobei der Prozessor (441 - 443) über den Sensor die anfänglichen Positionen der beiden Hände des Fahrers auf dem Lenkrad erkennt, bevor die Aktivierungsfunktion ausgeführt wird, und ferner die Händigkeit des Fahrers basierend darauf identifiziert, welche Hand die beleuchtete Position berührt.

5. Fahrzeug nach Anspruch **1**, wobei das ADAS einen Parameter der automatisierten Fahrzeugnavigationsvorgänge in Reaktion auf den vom Prozessor berechneten Grad der Bereitschaft des Fahrers konfiguriert.

6. Fahrzeug nach Anspruch **5,** wobei der Parameter ein Abstand zu einem vorausfahrenden Fahrzeug ist.

7. Fahrzeug nach Anspruch **5**, wobei der Parameter eine Fahrzeuggeschwindigkeit ist.

8. Fahrzeug nach Anspruch **1**, wobei das ADAS das Fahrzeug als Reaktion auf einen vom Prozessor berechneten niedrigen Grad der Bereitschaft des Fahrers zu einer Parkposition navigiert.

9. Fahrzeug nach Anspruch **1**, wobei der Prozessor (441 - 443) die Aktivierungsfunktion auf der Grundlage eines zuvor berechneten Grades der Bereitschaft des Fahrers konfiguriert.

10. Fahrzeug nach Anspruch **9**, wobei die Aktivierungsfunktion eine Beleuchtungsfarbe auf der Grundlage des zuvor berechneten Grades der Bereitschaft des Fahrers auswählt.

11. Fahrzeug nach Anspruch **1**, wobei das ADAS den Prozessor (441 - 443) anweist, die Aktivierungs-, Erkennungs- und Berechnungsfunktionen auf der Grundlage einer voraussichtlichen Änderung der Fahrbedingungen durchzuführen.

12. Fahrzeug nach Anspruch **11**, wobei die voraussichtliche Änderung der Fahrbedingungen eine automatische Navigationsanweisung zum Verlassen einer Autobahn ist.

## Revendications

1. Véhicule, comprenant :
un système avancé d'aide à la conduite (ADAS) qui exécute sélectivement, et arrête sélectivement, les opérations de navigation automatisée du véhicule ;
un volant (410) avec lequel un conducteur humain dirige le véhicule lorsque les opérations de navigation automatisées ne sont pas exécutées ;
un capteur (414, 100, 200, 300, 440) capable de détecter la main du conducteur à plusieurs endroits du volant ;
une pluralité d'émetteurs de lumière visible (106 - 109) capables d'éclairer la pluralité d'emplacements sur ledit volant ; et
un processeur (441 - 443), couplé audit ADAS, audit capteur et auxdits émetteurs de lumière visible, exécutant les fonctions suivantes pendant que ledit ADAS effectue les opérations de navigation automatisée du véhicule :
(i) une fonction d'activation qui active au moins l'un desdits émetteurs pour éclairer un emplacement sur ledit volant,
(ii) une fonction de détection qui détecte, par l'intermédiaire dudit capteur, lorsque le conducteur touche l'endroit éclairé, et
(iii) une fonction de calcul qui calcule le degré de préparation du conducteur à prendre le contrôle de la direction du véhicule lorsque l'ADAS cesse d'exécuter les opérations de navigation automatisée, sur la base d'au moins un des éléments suivants : le temps écoulé entre la fonction d'activation et la fonction de détection, et la proximité de la touche du conducteur par rapport à l'endroit éclairé.

2. Véhicule selon la revendication **1**, comprenant en outre un système audio, dans lequel la fonction d'activation amène le système audio à fournir une invite audio au conducteur pour qu'il touche l'endroit illuminé.

3. Véhicule selon la revendication **1**, dans lequel ledit processeur (441 - 443) détecte, via ledit capteur, un emplacement initial de la main du conducteur sur ledit volant avant d'exécuter la fonction d'activation, et sélectionne un emplacement sur ledit volant à éclairer en fonction de l'emplacement initial détecté.

4. Véhicule selon la revendication **1**, dans lequel ledit processeur (441 - 443) détecte, via ledit capteur, les emplacements initiaux des deux mains du conducteur sur ledit volant avant d'exécuter la fonction d'activation, et identifie en outre la main du conducteur en fonction de la main qui touche l'emplacement éclairé.

5. Véhicule selon la revendication **1**, dans lequel ledit ADAS configure un paramètre des opérations de navigation automatisée du véhicule en réponse au degré de préparation du conducteur calculé par ledit processeur.

6. Véhicule selon la revendication **5,** dans lequel le paramètre est une distance par rapport à un véhicule précédent.

7. Véhicule selon la revendication **5**, dans lequel le paramètre est la vitesse du véhicule.

8. Véhicule selon la revendication **1**, dans lequel l'ADAS dirige le véhicule vers un emplacement de stationnement en réponse à un faible degré de préparation du conducteur calculé par le processeur.

9. Véhicule selon la revendication **1**, dans lequel ledit processeur (441 - 443) configure la fonction d'activation sur la base d'un degré de préparation du conducteur calculé au préalable.

10. Véhicule selon la revendication **9**, dans lequel la fonction d'activation sélectionne une couleur d'éclairage en fonction du degré de préparation du conducteur calculé au préalable.

11. Véhicule selon la revendication **1**, dans lequel l'ADAS demande au processeur (441 - 443) d'exécuter les fonctions d'activation, de détection et de calcul sur la base d'un changement anticipé des conditions de conduite.

12. Véhicule selon la revendication **11**, dans lequel le changement anticipé des conditions de conduite est une instruction de navigation automatisée pour quitter une autoroute.
